(19) Europäisches Patentamt
European Patent Office
Office européen des brevets

(11) **EP 1 330 455 B1**

(12) **EUROPÄISCHE PATENTSCHRIFT**

(45) Veröffentlichungstag und Bekanntmachung des Hinweises auf die Patenterteilung:
**03.08.2005 Patentblatt 2005/31**

(21) Anmeldenummer: **01988718.1**

(22) Anmeldetag: **25.10.2001**

(51) Int Cl.⁷: **C07D 471/04**, A61P 11/00, A61K 31/40

(86) Internationale Anmeldenummer:
**PCT/EP2001/012376**

(87) Internationale Veröffentlichungsnummer:
**WO 2002/034747 (02.05.2002 Gazette 2002/18)**

(54) **NEUE 7-AZAINDOLE, DEREN VERWENDUNG ALS INHIBITOREN DER PHOSPHODIESTERASE 4 UND VERFAHREN ZU DEREN HERSTELLUNG**

NOVEL 7-AZAINDOLES, USE THEREOF AS PHOSPHODIESTERASE 4 INHIBITORS AND METHOD FOR PRODUCING THE SAME

NOUVEAUX 7-AZAINDOLES, LEUR UTILISATION EN TANT QU'INHIBITEURS DE LA PHOSPHODIESTERASE 4 ET LEUR PROCEDE DE PRODUCTION

(84) Benannte Vertragsstaaten:
**AT BE CH CY DE DK ES FI FR GB GR IE IT LI LU MC NL PT SE TR**
Benannte Erstreckungsstaaten:
**LT LV MK RO SI**

(30) Priorität: **27.10.2000 DE 10053275**
**30.10.2000 US 244342 P**

(43) Veröffentlichungstag der Anmeldung:
**30.07.2003 Patentblatt 2003/31**

(73) Patentinhaber: **Elbion AG**
**01445 Radebeul (DE)**

(72) Erfinder:
- **HÖFGEN, Norbert**
  **01458 Ottendorf-Okrilla (DE)**
- **EGERLAND, Ute**
  **01445 Radebeul (DE)**
- **KRONBACH, Thomas**
  **01445 Radebeul (DE)**
- **MARX, Degenhard**
  **78315 Radolfzell (DE)**
- **SZELENYI, Stefan**
  **90571 Schwaig (DE)**
- **KUSS, Hildegard**
  **01109 Dresden (DE)**
- **POLYMEROPOULOS, Emmanuel**
  **60325 Frankfurt am Main (DE)**

(74) Vertreter: **Dey, Michael, Dr. et al**
**Weickmann & Weickmann,**
**Patentanwälte,**
**Postfach 86 08 20**
**81635 München (DE)**

(56) Entgegenhaltungen:
**WO-A-96/11929** **GB-A- 1 141 949**
**JP-A- 10 120 681**

- **MOUADDIB, ABDERRAHIM ET AL: "Synthesis of indolo[3,2-c]quinoline and pyrido[3',2':4,5]pyrrolo[3,2- c]quinoline derivatives" SYNTHESIS (2000), (4), 549-556 , XP002189398**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 330 455 B1

**Beschreibung**

Technisches Gebiet

Die Erfindung betrifft substituierte 7-Azaindole der allgemeinen Formel ,

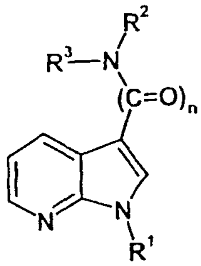

1

Verfahren zu deren Herstellung, pharmazeutische Zubereitungen, die diese Verbindungen enthalten sowie die pharmazeutische Verwendung dieser Verbindungen, die Inhibitoren der Phosphodiesterase 4 sind, als Wirkstoffe zur Behandlung von Erkrankungen, die mit einer Hemmung der Phosphodiesterase 4 - Aktivität in immunkompetenten Zellen (z.B. Makrophagen und Lymphozyten) durch die erfindungsgemäßen Verbindungen zu beeinflussen sind.

Stand der Technik

Die Aktivierung von Rezeptoren der Zellmembran durch Transmitter führt zur Aktivierung des "second messenger"-Systems. Die Adenylatcyclase synthetisiert aus AMP und GMP das wirksame cyclische AMP (cAMP) bzw. cyclische GMP (cGMP). Diese führen z.B. in glatten Muskelzellen zur Erschlaffung bzw. in Entzündungszellen zur Hemmung der Mediatorfreisetzung bzw. -synthese. Der Abbau der "second messenger" cAMP und cGMP erfolgt durch die Phosphodiesterasen (PDE). Bisher sind 11 Familien von PDE-Enzymen (PDE1-11) bekannt, die sich durch ihre Substratspezifität (cAMP, cGMP oder beides) und die Abhängigkeit von anderen Substraten (z.B. Calmodulin) unterscheiden. Diese Isoenzyme besitzen unterschiedliche Funktionen im Körper und sind in den einzelnen Zellarten unterschiedlich ausgeprägt (Beavo JA, Conti M and Heaslip RJ. Multiple cyclic nucleotide phosphodiesterases. Mol. Pharmacol. 1994, 46:399-405; Hall IP. Isoenzyme selective phosphodiesterase inhibitors: potential clinical uses, Br. J. clin. Pharmacol. 1993, 35:1-7). Durch Hemmung der verschiedenen PDE Isoenzymtypen kommt es zu einer Kumulation von cAMP bzw. cGMP in den Zellen, was therapeutisch genutzt werden kann (Torphy TJ, Livi GP, Christensen SB. Novel Phosphodiesterase Inhibitors for the Therapy of Asthma, Drug News and Perspectives 1993, 6:203-214).

In den für allergische Entzündungen wichtigen Zellen (Lymphozyten, Mastzellen, eosinophile Granulozyten, Makrophagen) ist das vorherrschende PDE-Isoenzym der Typ 4 (Torphy, J T. and Undem, B. J. Phosphordiesterase inhibitors: new opportunities for the treatment of asthma. Thorax 1991, 46:512-523). Die Hemmung der PDE 4 durch geeignete Inhibitoren wird daher als wichtiger Ansatz zur Therapie einer Vielzahl allergisch induzierter Erkrankungen betrachtet (Schudt Ch, Dent G, Rabe K Phosphodiesterase Inhibitors, Academic Press London 1996).

Eine wichtige Eigenschaft von Phosphodiesterase 4 Inhibitoren ist die Hemmung der Freisetzung von Tumornekrosefaktor $\alpha$ (TNF$\alpha$) aus Entzündungszellen. TNF$\alpha$ ist ein bedeutendes pro-inflammatorisches Cytokin, das eine Vielzahl biologischer Prozesse beeinflusst. Freigesetzt wird TNF$\alpha$ zum Beispiel aus aktivierten Macrophagen, aktivierten T-Lymphozyten, Mastzellen, Basophilen, Fibroblasten, Endothelzellen und Astrozyten im Gehirn. Es wirkt selbst aktivierend auf Neutrophile, Eosinophile, Fibroblasten und Endothelzellen, wodurch verschiedene gewebezerstörende Mediatoren freigesetzt werden. In Monozyten, Macrophagen und T-Lymphozyten bewirkt TNF$\alpha$ die vermehrte Produktion von weiteren proinflammatorischen Cytokinen wie GM-CSF (Granulocy-macrophage colony-stimulating factor) oder Interleukin-8. Auf Grund seiner entzündungsfördernden und katabolischen Wirkung spielt TNF$\alpha$ bei einer Vielzahl von Erkrankungen, wie Entzündungen der Atemwege, Entzündungen der Gelenke, endotoxischer Schock, Gewebsabstoßungen, AIDS und zahlreichen anderen immunologischen Erkrankungen eine zentrale Rolle. Für die Therapie solcher mit TNF$\alpha$ verbundener Erkrankungen sind Inhibitoren der Phosphodiesterase 4 somit ebenfalls geeignet.

Chronisch obstruktive Lungenerkrankungen (chronic obstructive pulmonary diseases, COPD) sind in der Bevölkerung weit verbreitet und haben auch eine große ökonomische Bedeutung. So verursachen COPD-Erkrankungen ca. 10-15 % aller Krankheitskosten in den entwickelten Ländern und ca. 25 % aller Todesfälle in den USA sind auf diese Ursache zurückzuführen (Norman P.: COPD: New developments and therapeutic opportunities, Drug News Perspect. 11 (7), 431-437, 1998), allerdings sind die Patienten zum Todeszeitpunkt meist über 55 Jahre alt (Nolte D.:

Chronische Bronchitis - eine Volkskrankheit multifaktorieller Genese. Atemw.-Lungenkrkh. 20 (5), 260-267, 1994). Die WHO schätzt ein, dass COPD innerhalb der nächsten 20 Jahre die dritthäufigste Todesursache sein wird.

Unter dem Krankheitsbild der chronisch obstruktiven Lungenerkrankungen (COPD) werden verschiedene Krankheitsbilder von chronischen Bronchitiden mit den Symptomen Husten und Auswurf sowie fortschreitender und irreversibler Verschlechterung der Lungenfunktion (besonders betroffen ist die Expiration) zusammengefasst. Der Krankheitsverlauf ist schubförmig und oft durch bakterielle Infektionen kompliziert (Rennard S. I.: COPD: Overview of definitions, Epidemiology, and factors influencing its development. Chest, 113 (4) Suppl., 235S-241S, 1998). Im Verlauf der Erkrankung nimmt die Lungenfunktion stetig ab, die Lunge wird zunehmend emphysematös und die Atemnot der Patienten wird offensichtlich. Diese Erkrankung beeinträchtigt deutlich die Lebensqualität der Patienten (Kurzatmigkeit, geringe Belastbarkeit) und verkürzt signifikant deren Lebenserwartung. Der Hauptrisikofaktor neben Umweltfaktoren ist das Rauchen (Kummer F.: Asthma und COPD. Atemw.-Lungenkrkh. 20 (5), 299-302, 1994; Rennard S. I.: COPD: Overview of definitions, Epidemiology, and factors influencing its development. Chest, 113 (4) Suppl., 235S-241S, 1998) und daher sind Männer deutlich häufiger betroffen, als Frauen. Durch die Veränderung der Lebensgewohnheiten und den Anstieg der Anzahl der Raucherinnen wird sich dieses Bild jedoch in Zukunft verschieben.

Die gegenwärtige Therapie zielt nur auf die Linderung der Symptome, ohne ursächlich in die Progression der Erkrankung einzugreifen. Der Einsatz von langwirkenden Beta2-Agonisten (z.B. Salmeterol) evtl. in Kombination mit muscarinergen Antagonisten (z. B. Ipratropium) verbessert die Lungenfunktion durch Bronchodilatation und wird routinemäßig eingesetzt (Norman P.: COPD: New developments and therapeutic opportunities, Drug News Perspect. 11 (7), 431-437, 1998). Eine große Rolle bei den COPD-Schüben spielen bakterielle Infektionen, die mit Antibiotika behandelt werden müssen (Wilson R.: The role of infection in COPD, Chest, 1 13 (4) Suppl., 242S-248S, 1998; Grossman R. F.: The value of antibiotics and the outcomes of antibiotic therapy in exacerbations of COPD. Chest, 113 (4) Suppl., 249S-255S, 1998). Die Therapie dieser Erkrankung ist bisher noch unbefriedigend, besonders im Hinblick auf die stetige Abnahme der Lungenfunktion. Neue Therapieansätze, die an Entzündungsmediatoren, Proteasen oder Adhäsionsmolekülen angreifen, könnten sehr erfolgversprechend sein (Barnes P.J.: Chronic obstructive disease: new opportunities for drug development, TiPS 10 (19), 415-423, 1998).

Unabhängig von den die Erkrankung komplizierenden bakteriellen Infektionen findet man in den Bronchien eine chronische Entzündung, welche durch neutrophile Granulozyten dominiert wird. Für die beobachteten strukturellen Veränderungen in den Atemwegen (Emphysem) werden unter anderem die durch neutrophile Granulozyten freigesetzten Mediatoren und Enzyme verantwortlich gemacht. Die Hemmung der Aktivität der neutrophilen Granulozyten ist somit ein rationaler Ansatz, um ein Fortschreiten der COPD (Verschlechterung der Lungenfunktionparameter) zu verhindern oder zu verlangsamen. Ein wichtiger Stimulus für die Aktivierung der Granulozyten ist das pro-inflammatorische Cytokin TNFα (tumour necrosis factor). So ist bekannt, dass TNFα die Bildung von Sauerstoff-Radikalen durch neutrophile Granulozyten stimuliert (Jersmann, H.P.A.; Rathjen, D.A. and Ferrante A.: Enhancement of LPS-induced neutrophil oxygen radical production by TNFα, Infection and Immunity, 4, 1744-1747, 1998). PDE4-Inhibitoren können sehr wirksam die Freisetzung von TNFα aus einer Vielzahl von Zellen hemmen und somit die Aktivität der neutrophilen Granulozyten unterdrücken. Der unspezifische PDE-Inhibitor Pentoxifylline ist in der Lage, sowohl die Bildung von Sauerstoff-Radikalen als auch die Phagozytosefähigkeit von neutrophilen Granulozyten zu hemmen (Wenisch, C.; Zedtwitz-Liebenstein, K.; Parschalk, B. and Graninger W.: Effect of pentoxifylline in vitro on neutrophil reactive oxygen production and phagocytic ability assessed by flow cytometry, Clin. Drug Invest., 13(2):99-104, 1997).

Es sind bereits verschiedene PDE 4 Inhibitoren bekannt. Vorrangig handelt es sich dabei um Xanthin-Derivate, Rolipram-Analoge oder Nitraquazon-Abkömmlinge (Übersicht in: Karlsson J-A, Aldos D Phosphodiesterase 4 inhibitors for the treatment of asthma, Exp. Opin. Ther. Patents 1997, 7: 989-1003). Keine dieser Verbindungen konnte bisher bis zur klinischen Anwendung gebracht werden. Es musste festgestellt werden, dass die bekannten PDE 4 Inhibitoren auch verschiedene Nebenwirkungen wie Nausea und Emesis besitzen, die bisher nicht ausreichend zurückgedrängt werden konnten. Deshalb ist die Entdeckung neuer PDE 4 Inhibitoren mit besserer therapeutischer Breite erforderlich.

Die Verwendung von 7-Azaindolen bei der Entwicklung neuer Wirkstoffe für verschiedene Indikationen ist bisher nur in relativ wenigen Fällen beschrieben.

In der japanischen Patentschrift JP 10120681 (Fujisawa Pharmaceutical Co., Ltd.) werden 5- und 7- Azaindole der allgemeinen Formel

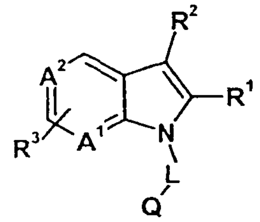

beansprucht, wobei $R^1$ für Wasserstoff oder kurze Alkyl-Gruppen steht, $R^2$ Wasserstoff, Halogen, kurze Alkyl-Gruppen, Cycloalkyl-Gruppen, Alkylcarbonyl-Gruppen oder Alkanoyl-Gruppen bedeuten kann, $R^3$ für Alkanoyl-Gruppen, geschützte Carbonsäure-Gruppen, die Cyano-Gruppe oder substituierte Carbamoyl-Gruppen steht. L bedeutet eine kurze Alkylen-Brücke. Q steht für substituierte Aromaten und Heterocyclen. Von $A^1$ und $A^2$ steht je einer für N und der andere für CH. Diese Verbindungen unterscheiden sich von den erfindungsgemäßen Verbindungen insbesondere bezüglich der Substituenten $R^2$ und $R^3$, teilweise in $R^1$ und $A^2$. Die beschriebenen Verbindungen werden als Inhibitoren einer cGMP spezifischen Phosphodiesterase (PDE 5) beansprucht. Als Anwendungsgebiete werden verschiedene Herz-Kreislauferkrankungen, Bronchitis, Asthma, Rhinitis, Impotenz, diabetische Komplikationen und Glaucom benannt.

Von L.N. Yakhontov, S.S. Liberman, D.M. Krasnokutskaya et al. werden in Khim.-Farm. Zh. 8 (11), 1974, 5-9 die Synthesen verschiedener 3-Aminoalkyl-4-azaindole und 3-Aminoalkyl-7-azaindole beschrieben. Für 3-(2-Aminoethyl)-7-azaindole wird depressive oder antidepressive Wirkung beschrieben. Für 3-Aminomethyl-7-azaindole wurde eine blutdrucksenkende Wirkung festgestellt.

A.J. Verbiscar beschreibt in J. Med. Chem. 15 (2), 1972, 149-52 die Verbindung der Formel

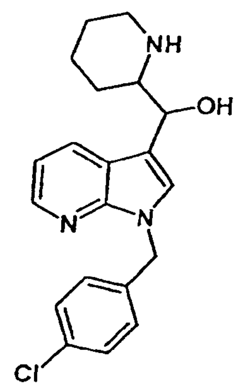

für die eine Antimalaria-Wirkung bestimmt wurde.

WO 96/11929 beschreibt 5 HT 2C/2B-Antagonisten, welche zur Behandlung von Erkrankungen des ZNS einsetzbar sind.

In der Patentschrift US 650223 (Sterling Drug Inc.) wird die Synthese verschiedener 2-(Imidazolin-2-yl)-alkyl-7-azaindole bzw. 3-(Imidazolin-2-yl)-alkyl-7-azaindole aus den entsprechenden 2- oder 3-Cyanoalkyl-7-azaindolen beschrieben und für diese Verbindungen eine Anwendung als Vasokonstriktoren beansprucht.

Als Inhibitoren der PDE 4 sind 7-Azaindole bisher völlig unbekannt.

<u>Beschreibung der Erfindung</u>

Die Erfindung betrifft substituierte 7-Azaindole der allgemeinen:Formel 1 ,

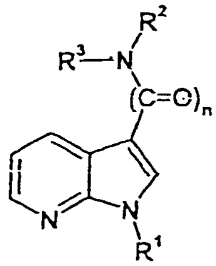

1

worin

n = 1 oder 2 sein kann, und

$R^1$ für

$-C_{1...10}$-Alkyl, geradkettig oder verzweigtkettig, unsubstituiert oder ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NO_2$, -CN, -F, -Cl, -Br, -I, $-O-C_{1...6}$-Alkyl, $-O-C_{6...14}$-Aryl, $-S-C_{1...6}$-Alkyl, $-S-C_{6...14}$Aryl, $-SO_3H$, $-SO_2C_{1...6}$Alkyl, $-SO_2C_{6...14}$Aryl, $-OSO_2C_{1...6}$Alkyl, $-OSO_2C_{6...14}$Aryl, -COOH, $-(CO)C_{1...5}$Alkyl, mit mono-, bi- oder tricyclischen gesättigten oder ein- oder mehrfach ungesättigten Carbocyclen mit 3...14 Ringgliedern, mit mono-, bi- oder tricyclischen gesättigten oder ein- oder mehrfach ungesättigten Heterocyclen mit 5...15 Ringgliedern und 1...6 Heteroatomen, die vorzugsweise N, O und S sind, wobei die $C_{6...14}$Aryl-Gruppen und die carbocyclischen und heterocyclischen Substituenten ihrerseits unsubstituiert oder ein- oder mehrfach mit $R^4$ substituiert sein können,

$-C_{2...10}$-Alkenyl, ein- oder mehrfach ungesättigt, geradkettig oder verzweigtkettig, unsubstituiert, oder ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NO_2$, -CN, -F, -Cl, -Br, -I, $-O-C_{1...6}$-Alkyl, $-O-C_{6...14}$-Aryl, $-S-C_{1...6}$-Alkyl, $-S-C_{6...14}$Aryl, $-SO_3H$, $-SO_2C_{1...6}$Alkyl, $-SO_2C_{6...14}$Aryl, $-OSO_2C_{1...6}$Alkyl, $-OSO_2C_{6...14}$Aryl, -COOH, $-(CO)C_{1...5}$Alkyl, mit mono-, bi- oder tricyclischen gesättigten oder ein- oder mehrfach ungesättigten Carbocyclen mit 3 ...14 Ringgliedern, mit mono-, bi- oder tricyclischen gesättigten oder ein- oder mehrfach ungesättigten Heterocyclen mit 5...15 Ringgliedern und 1...6 Heteroatomen, die vorzugsweise N, O und S sind, wobei die $C_{6...14}$Aryl-Gruppen und die carbocyclischen und heterocyclischen Substituenten ihrerseits unsubstituiert oder ein- oder mehrfach mit $R^4$ substituiert sein können, steht,

$R^2$ und $R^3$ gleich oder verschieden sein können, wobei nur einer von beiden für Wasserstoff stehen kann und $R^2$ und $R^3$ weiterhin $-C_{1...5}$-Alkyl, unsubstituiert

oder ein- oder mehrfach substituiert mit -OH, -SH, $-NH_2$, $-NHC_{1...6}$-Alkyl, $-N(C_{1...6}\text{-Alkyl})_2$, $-NO_2$, -CN, -F, -Cl, -Br, -I, $-O-C_{1...6}$-Alkyl, $-S-C_{1...6}$-Alkyl, -Phenyl, -Pyridyl,

-Phenyl, unsubstituiert oder ein- oder mehrfach substituiert mit -OH, -SH, $-NH_2$, $-NHC_{1...3}$-Alkyl, $-N(C_{1...3}\text{-Alkyl})_2$, $-NO_2$, -CN, -COOH, $-COOC_{1...3}$Alkyl, -F, -Cl, -Br, $-O-C_{1...3}$-Alkyl, $-S-C_{1...3}$-Alkyl,

-Pyridyl, unsubstituiert oder ein- oder mehrfach substituiert mit $-NO_2$, -CN, -COOH, $-COOC_{1...3}$Alkyl, -Cl, -Br, $-O-C_{1...3}$-Alkyl, $-S-C_{1...3}$-Alkyl,

sowie

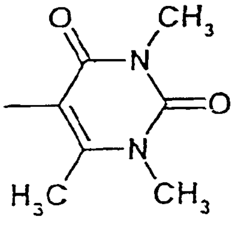
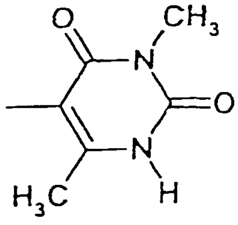
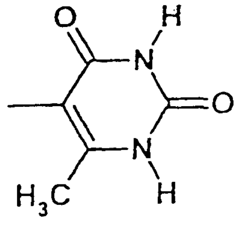
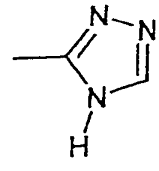

bedeuten können,

weiterhin die Gruppe $-NR^2R^3$ zusammen für

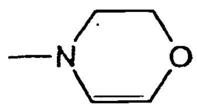 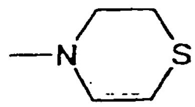 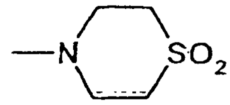 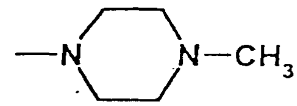

stehen kann,

und

$R^4$ für

-H, -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NHCOC_{1...6}$Alkyl, $-NO_2$, -CN, -COOH, $-COOC_{1...6}$Alkyl, $-(CO)C_{1...6}$Alkyl, $-(CS)C_{1...6}$Alkyl, -F, -Cl, -Br, -I, $-O-C_{1...6}$-Alkyl, $-O-C_{6...14}$-Aryl, $-S-C_{1...6}$-Alkyl, $-S-C_{6...14}$Aryl, $-SOC_{1...6}$Alkyl, $-SO_2C_{1...6}$Alkyl steht,

mit der Maßgabe, dass, wenn n = 1 nicht gleichzeitig

$R_1 = -C_{1...6}$-Alkyl,

$R_2$ = -H oder $-C_{1...6}$-Alkyl und

$R_3$ =

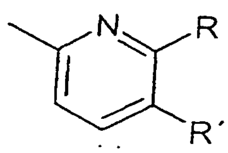 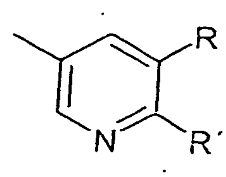 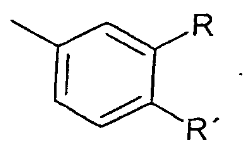

wobei R, R' unabhängig voneinander $-C_{1...10}$-Alkyl, gegebenenfalls ein- oder mehrfach mit Halogen substituiert, $C_{2...6}$-Alkenyl, $-C_{3...6}$Cycloalkyloxy, $C_{3...6}$Cycloalkyl$C_{1...6}$alkoxy, $C_{2...6}$Alkinyl, $C_{3...6}$Cycloalkyl, $-C_{3...6}$-Cycloalkyl$C_{1...6}$alkyl, $-C_{1...6}$Alkylthio, $-C_{3...6}$Cycloalkyl$C_{1...6}$salkylthio, $-C_{1...6}$Alkoxy, Hydroxy-, Halogen-, Nitro-, $-CF_3$, $-C_2F_5$, $-OCF_3$, $-SCF_3$, $-SO_2CF_3$, $-SO_2F$, Formyl-, $-C_{2...6}$-Alkanoyl, Cyano-, gegebenenfalls substituiertes Phenyl- oder Thienyl, $-NR''_2$, $-CONR''_2$, -COOR" sind

oder

R + R' zusammen ein 5-gliedriger Carbocyklus oder Heterocyklus sind,

und

R" = -H oder $-C_{1...6}$Alkyl.

In den erfindungsgemäßen 7-Azaindolen der Formel 1 ist der Rest $R^1$ bevorzugt ein $C_1$-$C_{10}$-Alkylrest. Ein solcher Alkylrest kann linear, verzweigt oder cyclisch sein und ist bevorzugt linear. Besonders bevorzugt sind Alkylreste mit 1 bis 6, noch mehr bevorzugt mit 1 bis 4 Kohlenstoffatomen. In einer weiteren bevorzugten Ausführungsform steht $R^1$ für einen $C_2$-$C_{10}$, insbesondere einen $C_2$-$C_6$ und am meisten bevorzugt einen $C_2$-$C_4$-Alkenylrest. Der Alkenylrest kann ein- oder mehrfach, beispielsweise zwei- oder dreifach ungesättigt sein. Bei dem Alkenylrest kann es sich um einen geraden, verzweigten oder cyclischen Kohlenwasserstoffrest handeln. Besonders bevorzugt sind Reste $R^1$ in denen der Alkyl- oder Alkenylrest ein- oder mehrfach, beispielsweise zweifach, dreifach, vierfach oder fünffach substituiert ist. Besonders bevorzugt ist der Rest $R^1$ ein substituierter $C_1$-Alkyl- (also Methyl-) Rest. Von den oben angegebenen Substituenten für die Alkyl- bzw. Alkenylgruppe des Restes $R^1$ sind insbesondere bevorzugt die Subsituenten -OH, -F, -Cl, -Br, -I, $-C_1$-$C_4$-Alkoxy. Weiterhin sind Substituenten bevorzugt, in denen ein gegebenenfalls vorhandener Alkylrest 1 bis 4 Kohlenstoffatome und ein gegebenenfalls vorhandener Arylrest 6 bis 10 Kohlenstoffatome aufweist. Von den Carbocylen bevorzugt ist der Phenylrest, insbesondere ein substituierter Phenylrest, welcher bevorzugt mit -F, -Cl, -Br, -I, $C_1$-$C_6$-Alkoxy oder Hydroxy substituiert ist. Von den Heterocyclen sind solche bevorzugt, die mindestens ein Heteroatom ausgewählt aus N, O oder S aufweisen. Besonders bevorzugt von den Heterocyclen ist der Pyridylrest sowie der Isoxazolrest, insbesondere der 3,5-Dimethylisooxazolrest. Ein Beispiel für einen kondensierten carbocyclischen Substituenten ist der Naphthylrest.

Besonders bevorzugt steht $R^1$ für eine Gruppierung, welche einen cyclischen Kohlenwasserstoffrest umfasst, wie etwa für Cyclopropylmethyl, für einen linearen Kohlenwasserstoff, wie etwa n-Hexyl, für einen mit einem Alkoxyrest substituierten linearen Kohlenwasserstoff, wie etwa 2-Methoxyethyl, für einen verzweigten Kohlenwasserstoffrest, wie etwa Isobutyl, für einen ungesättigten Kohlenwasserstoffrest, wie etwa für 2-Methylpropen-3-yl oder für einen eine aromatische Gruppe enthaltenen Kohlenwasserstoffrest, welcher gegebenenfalls substituiert sein kann, wie etwa für

4-Fluorbenzyl, 3-Methoxybenzyl, 4-Methoxybenzyl, 4-Chlorbenzyl, 4-Methylbenzyl, 3-Hydroxybenzyl oder 4-Hydroxybenzyl, für eine einen heteroaromatischen Kohlenwasserstoff enthaltene Gruppe, wie etwa für 4-Pyridylmethyl oder 3,5-Dimethylisoxaxol-4-methyl oder für eine einen kondensierten aromatischen Kohlenwasserstoff enthaltene Gruppe, wie etwa 1-Naphthylmethyl.

Die Substituenten am Stickstoffatom, $R^2$ und $R^3$ können in einer bevorzugten Ausführungsform einen gegebenenfalls substituierten $C_1$-$C_5$, insbesondere $C_1$-$C_3$ und besonders bevorzugt $C_1$ (entspricht Methyl)-Alkylrest darstellen.

Bevorzugt bedeutet einer der Reste $R^2$ oder/und $R^3$ einen Rest, welcher einen heteroaromatischen Kohlenwasserstoff umfasst, wie etwa 4-Pyridylmethyl, wobei der heteroaromatische Kohlenwasserstoff weiterhin substituiert sein kann, bevorzugt mit einem Halogen, wie etwa 3,5-Dichlor-4-pyridyl. In einer weiteren bevorzugten Ausführungsform handelt es sich bei $R^2$ oder/und $R^3$ um den Rest Morpholino. Weiterhin bevorzugt sind Reste $R^2$ und $R^3$, welche einen aromatischen Kohlenwasserstoff umfassen, welcher bevorzugt substituiert ist, insbesondere mit Halogen oder Carboxy, wie etwa 2,6-Dichlorphenyl, 4-Carboxyphenyl, 4-Ethoxycarbonylphenyl, 3,4-Dimethoxyphenyl. Weiterhin bevorzugt bedeuten sowohl $R^2$ als auch $R^3$ Methoxyethyl. In einer weiteren bevorzugten Ausführungsform steht $R^2$ oder $R^3$ für einen Rest

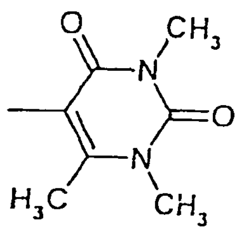
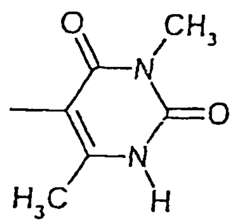
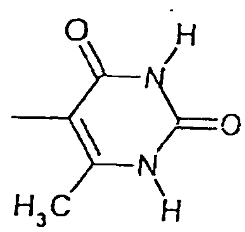
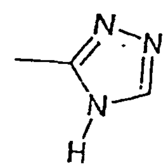

oder die Gruppe $-NR^2R^3$ zusammen für

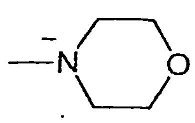
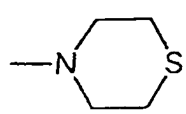
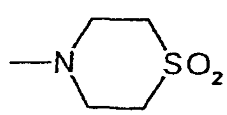
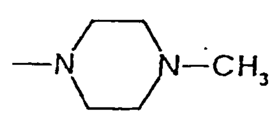

Bevorzugte Verbindungen der Formel I, worin n = 1 ist, sind
N-(4-Pyridylmethyl)-1-cyclopropylmethyl-7-azaindol-3-carbonsäureamid,
N-(3,5-Dichlorpyridin-4-yl)-1-isobutyl-7-azaindol-3-carbonsäureamid,
N-(3,5-Dichlorpyridin-4-yl)-1-hexyl-7-azaindol-3-carbonsäureamid,
N-(3,5-Dichlorpyridin-4-yl)-1-cyclopropylmethyl-7-azaindol-3-carbonsäureamid,
N-(4-Pyridylmethyl)-1-(4-fluorbenzyl)-7-azaindol-3-carbonsäureamid,
N-(3,5-Dichlorpyridin-4-yl)-1-(4-fluorbenzyl)-7-azaindol-3-carbonsäureamid,
N-(3,5-Dichlorpyridin-4-yl)-1-(4-methoxybenzyl)-7-azaindol-3-carbonsäureamid,
N-(4-Pyridylmethyl)-1-(4-chlorbenzyl)-7-azaindol-3-carbonsäureamid,
1-(4-Fluorbenzyl)-7-azaindol-3-carbonsäuremorpholid,
N-(2,6-Dichlorphenyl)-1-(2-methylpropen-3-yl)-7-azaindol-3-carbonsäureamid und
N-(3, 5-Dichlorpyridin-4-yl)-1-(4-pyridylmethyl)-7-azaindol-3-carbonsäureamid.

Bevorzugte Verbindungen der Formel)I, worin n = 2 ist, sind
N-(3,5-Dichlorpyridin-4-yl)-[1-(3-methoxybenzyl)-7-azaindol-3-yl]-glyoxylsäureamid,
N-(4-Pyridyl)-[1-(4-fluorbenzyl)-7-azaindol-3-yl]-glyoxylsäureamid-hydrochlorid,
N-(3,5-Dichlorpyridin-4-yl)-[1-(4-fluorbenzyl)-7-azaindol-3-yl]-glyoxylsäureamid,
N-(4-Pyridyl)-[1-(4-chlorbenzyl)-7-azaindol-3-yl]-glyoxylsäureamid-hydrochlorid,
N-(3,5-Dichlorpyridin-4-yl)-[1-(4-chlorbenzyl)-7-azaindol-3-yl]-glyoxylsäureamid,
N-(3,5-Dichlorpyridin-4-yl)-[1-(4-methoxybenzyl)-7-azaindol-3-yl]-glyoxylsäureamid,
N-(2,6-Dichlorphenyl)-[1-(4-chlorbenzyl)-7-azaindol-3-yl]-glyoxylsäureamid,
N-(4-Carboxyphenyl)-[1-(4-fluorbenzyl)-7-azaindol-3-yl]-glyoxylsäureamid,
N-(4-Ethoxycarbonylphenyl)-[1-(4-fluorbenzyl)-7-azaindol-3-yl]-glyoxylsäureamid,

N-(3,4-Dimethoxyphenyl)-[1-(4-fluorbenzyl)-7-azaindol-3-yl]-glyoxylsäureamid,
N-(3,5-Dichlorpyridin-4-yl)-[1-(4-methylbenzyl)-7-azaindol-3-yl]-glyoxylsäureamid,
N-(3,5-Dichlorpyridin-4-yl)-[1-(4-hydroxybenzyl)-7-azaindol-3-yl]-glyoxylsäureamid,
N-(3,5-Dichlorpyridin-4-yl)-[1-(3-hydroxybenzyl)-7-azaindol-3-yl]-glyoxylsäureamid,
N-(3,5-Dichlorpyridin-4-yl)-(1-cyclopropylmethyl-7-azaindol-3-yl)-glyoxylsäureamid,
N-(3,5-Dichlorpyridin-4-yl)-(1-hexyl-7-azaindol-3-yl)-glyoxyl-säureamid,
N-(3, 5-Dichlorpyridin-4-yl)-(1-isobutyl-7-azaindol-3-yl)-glyoxyl-säureamid,
N-(3,5-Dichlorpyridin-4-yl)-[1-(2-methylpropen-3-yl)-7-azaindol-3-yl]-glyoxylsäureamid,
N-(3,5-Dichlorpyridin-4-yl)-[1-(2-methoxyethyl)-7-azaindol-3-yl]-glyoxylsäureamid,
N-(3,5-Dichlorpyridin-4-yl)-[1-(1-naphthylmethyl)-7-azaindol-3-yl]-glyoxylsäureamid,
N-(3,5-Dichlorpyridin-4-yl)-[1-(4-pyridylmethyl)-7-azaindol-3-yl]-glyoxylsäureamid,
N-(3, 5-Dichlorpyridin-4-yl)-[1-(3,5-dimethylisoxazol-4-ylmethyl)-7-azaindol-3-yl]-glyoxylsäureamid,
N,N-Bis(2-methoxyethyl)-[1-(4-fluorbenzyl)-7-azaindol-3-yl]-glyoxylsäureamid,
[1-(4-Fluorbenzyl)-7-azaindol-3-yl]-glyoxylsäuremorpholid,
[1-(4-Fluorbenzyl)-7-azaindol-3-yl]-glyoxylsäure-(S,S-dioxo-thiomorpholid),
[1-(4-Fluorbenzyl)-7-azaindol-3-yl]-glyoxylsäure-(4-methyl-piperazid),
N-(6-Methyluracil-5-yl)-[1-(4-fluorbenzyl)-7-azaindol-3-yl]-glyoxylsäureamid,
N-(3,6-Dimethyluracil-5-yl)-[1-(4-fluorbenzyl)-7-azaindol-3-yl]-glyoxylsäureamid,
N-(1,3,6-Trimethyluracil-5-yl)-[1-(4-fluorbenzyl)-7-azaindol-3-yl]-glyoxylsäureamid und
N-(1,2,4-4H-triazol-3-yl)-[1-(4-fluorbenzyl)-7-azaindol-3-yl]-glyoxylsäureamid.

Eine besonders bevorzugte Verbindung der Formel I ist
N-(35-Dichlorpyridin-4-yl)-[1-(4-fluorbenzyl)-7-azaindol-3yl]glyoxylsäureamid.

Weiterhin betrifft die Erfindung die physiologisch verträglichen Salze der Verbindungen gemäß Formel 1.

Die physiologisch verträglichen Salze werden in üblicher Weise durch Neutralisation der Basen mit anorganischen oder organischen Säuren bzw. durch Neutralisation der Säuren mit anorganischen oder organischen Basen erhalten. Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäure wie Essigsäure, Weinsäure, Milchsäure, Propionsäure, Glykolsäure, Malonsäure, Maleinsäure, Fumarsäure, Gerbsäure, Succinsäure, Alginsäure, Benzoesäure, 2-Phenoxybenzoesäure, 2-Acetoxybenzosäure, Zimtsäure, Mandelsäure, Zitronensäure, Apfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1 ,2-disulfonsäure, Benzolsulfonsäure, 4-Methylbenzolsulfonsäure oder Naphthalin-2-sulfonsäure in Frage. Als anorganische Basen kommen zum Beispiel Natronlauge, Kalilauge, Ammoniak sowie als organische Basen Amine, bevorzugt jedoch tertiäre Amine, wie Trimethylamin, Triethylamin, Pyridin, N,N-Dimethylanilin, Chinolin, Isochinolin, a-Picolin, b-Picolin, g-Picolin, Chinaldin oder Pyrimidin in Frage.

Des Weiteren können physiologisch verträgliche Salze der Verbindungen gemäß Formel 1 dadurch gewonnen werden, dass Derivate, die tertiäre Amino-Gruppen besitzen, in an sich bekannter Weise mit Quaternierungsmitteln in die entsprechenden quaternären Ammoniumsalze überführt werden. Als Quaternierungsmittel kommen beispielsweise Alkylhalogenide wie Methyliodid, Ethylbromid und n-Propylchlorid, aber auch Arylalkylhalogenide wie Benzylchlorid oder 2-Phenylethylbromid in Frage.

Weiterhin betrifft die Erfindung von den Verbindungen der Formel 1, die ein asymmetrisches Kohlenstoffatom enthalten, die D-Form, die L-Form und D,L-Mischungen sowie im Falle mehrerer asymmetrischer Kohlenstoffatome die diastereomeren Formen. Diejenigen Verbindungen der Formel 1, die asymmetrische Kohlenstoffatome enthalten und in der Regel als Razemate anfallen, können in an sich bekannter Weise beispielsweise mit einer optisch aktiven Säure in die optisch aktiven Isomeren getrennt werden. Es ist aber auch möglich, von vornherein eine optisch aktive Ausgangssubstanz einzusetzen, wobei dann als Endprodukt eine entsprechende optisch aktive beziehungsweise diastereomere Verbindung erhalten wird.

Für die erfindungsgemäßen Verbindungen wurden pharmakologisch bedeutende Eigenschaften gefunden, die therapeutisch genutzt werden können.

Die erfindungsgemäßen Verbindungen sind Inhibitoren der Freisetzung von TNF$\alpha$.

Die Verbindungen können deshalb zur Hemmung deer Freisetzung von TNF$\alpha$ eingesetzt werden.

Es ist daher Gegenstand dieser Erfindung, dass die Verbindungen gemäß Formel 1 und deren Salze sowie pharmazeutische Zubereitungen, die diese Verbindungen oder deren Salze enthalten, zur Behandlung von Erkrankungen verwendet werden können, bei denen eine Inhibition von TNF$\alpha$ nützlich ist.

Zu diesen Erkrankungen gehören beispielsweise Gelenksentzündungen einschließlich Arthritis und rheumatoide Arthritis sowie andere arthritische Erkrankungen wie rheumatoide Spondylitis und Osteoarthritis. Weitere Anwendungsmöglichkeiten sind die Behandlung von Patienten, die unter Osteoporose, Sepsis, septischem Schock, gramnegativer Sepsis, toxischem Schocksyndrom, Atemnotsyndrom, Asthma oder anderen chronischen pulmonalen Erkrankungen, Knochenresorptions-Krankheiten oder Transplantat-Abstoßungsreaktionen oder anderen Autoimmunerkrankungen, wie Lupus erythematosus, Multipler Sclerose, Glomerulonephritis und Uveitis, Insulin abhängigem Diabetes mellitus sowie chronischer Demyelinisierung leiden.

Außerdem können die erfindungsgemäßen Verbindungen auch zur Therapie von Infektionen wie Virusinfektionen und Parasiten-Infektionen, beispielsweise zur Therapie von Malaria, Leishmaniasis, infektionsbedingtem Fieber, infektions-bedingten Muskelschmerzen, AIDS und Kachexien eingesetzt werden.

Die erfindungsgemäßen Verbindungen sind Inhibitoren der Phosphodiesterase 4.

Die erfindungsgemäßen Verbindungen können deshalb zur Hemmung der Phosphodiesterase 4 eingesetzt werden.

Es ist daher Gegenstand dieser Erfindung, dass die Verbindungen gemäß Formel 1 und deren Salze sowie pharmazeutische Zubereitungen, die diese Verbindungen oder deren Salze enthalten, zur Behandlung von Erkrankungen verwendet werden können, bei denen eine Inhibition der Phosphodiesterase 4 nützlich ist.

So können die erfindungsgemäßen Verbindungen als Bronchodilatatoren und zur Asthma - Prophylaxe eingesetzt werden. Die Verbindungen gemäß Formel 1 sind weiterhin Inhibitoren der Akkumulation von Eosinophilen sowie deren Aktivität. Demzufolge können die erfindungsgemäßen Verbindungen auch bei Erkrankungen eingesetzt werden, bei denen Eosinophile eine Rolle spielen. Zu diesen Erkrankungen gehören beispielsweise entzündliche Atemwegserkrankungen wie Asthma bronchiale, allergische Rhinitis, allergische Konjunktivitis, atopische Dermatitis, Ekzeme, allergische Angiitis, durch Eosinophile vermittelte Entzündungen wie eosinophile Fasciitis, eosinophile Pneumonie und PIE-Syndrom (Pulmonale Infiltration mit Eosinophilie), Urtikaria, ulcerative Colitis, die Crohn-Krankheit und proliferative Hauterkrankungen wie Psoriasis oder Keratosis.

Gegenstand dieser Erfindung ist es, dass die Verbindungen gemäß Formel 1 und deren Salze sowohl Freisetzung von TNF$\alpha$ in vitro, als auch die LPS-induzierte pulmonale Neutrophilen-Infiltration bei Ratten in vivo inhibieren können. Die Gesamtheit dieser gefundenen pharmakologisch bedeutsamen Eigenschaften belegt, dass die Verbindungen gemäß Formel 1 und deren Salze sowie pharmazeutische Zubereitungen, die diese Verbindungen oder deren Salze enthalten, zur Behandlung von chronisch obstruktiven Lungenerkrankungen therapeutisch genutzt werden können.

Die erfindungsgemäßen Verbindungen besitzen weiterhin neuroprotektive Eigenschaften und können zur Therapie von Krankheiten verwendet werden, bei denen Neuroprotektion nützlich ist. Solche Erkrankungen sind beispielsweise senile Demenz (Alzheimer's Krankheit), Gedächtnisschwund, Parkinson's Krankheit, Depressionen, Schlaganfälle und Claudikatio intermittens.

Weitere Anwendungsmöglichkeiten der erfindungsgemäßen Verbindungen sind die Prophylaxe und Therapie von Prostata-Krankheiten, wie beispielsweise benigne Prostata-Hyperplasie, Pollakisurie, Nocturie sowie die Behandlung von Inkontinenz, von durch Harnsteine ausgelösten Koliken und von männlichen und weiblichen sexuellen Dysfunktionen.

Schließlich können die erfindungsgemäßen Verbindungen ebenfalls zur Inhibition der Entstehung einer Arzneimittelabhängigkeit bei wiederholtem Einsatz von Analgetika, wie beispielsweise Morphin sowie zur Verringerung der Toleranzentwicklung beim wiederholten Einsatz von diesen Analgetika verwendet werden.

Zur Herstellung der Arzneimittel wird neben den üblichen Hilfsmitteln, Träger- und Zusatzstoffen eine wirksame Dosis der erfindungsgemäßen Verbindungen oder deren Salze verwendet.

Die Dosierung der Wirkstoffe kann je nach Verabfolgungsweg, Alter, Gewicht des Patienten, Art und Schwere der zu behandelnden Erkrankungen und ähnlichen Faktoren variieren.

Die tägliche Dosis kann als einmal zu verabreichende Einzeldosis oder unterteilt in 2 oder mehrere Tagesdosen gegeben werden und beträgt in der Regel 0,001-100 mg .

Als Applikationsform bevorzugt sind orale, parenterale, intravenösen, transdermale, topische, inhalative und intranasel Zubereitungen.

Zur Anwendung kommen die üblichen galenischen Zubereitungsformen wie Tabletten, Dragees, Kapseln, dispergierbare Pulver, Granulate, wässrige Lösungen, wässrige oder ölige Suspensionen, Sirup, Säfte oder Tropfen.

Feste Arzneiformen können inerte Inhalts- und Trägerstoffe enthalten, wie z. B. Calciumcarbonat, Calciumphosphat, Natriumphosphat, Lactose, Stärke, Mannit, Alginate, Gelatine, Guar-Gummi, Magnesium- oder Aluminiumstearat, Methylcellulose, Talkum, hochdisperse Kieselsäuren, Silikonöl, höhermolekulare Fettsäuren (wie Stearinsäure), Gelatine, Agar-Agar oder pflanzliche oder tierische Fette und Öle, feste hochmolekulare Polymere (wie Polyethylenglykol); für orale Applikation geeignete Zubereitungen können gewünschtenfalls zusätzliche Geschmacks- und/oder Süßstoffe enthalten.

Flüssige Arzneiformen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze, Zucker oder Zuckeralkohole zur Regelung des osmotischen Drucks oder zur Pufferung und/oder Viskositätsregulatoren enthalten.

Derartige Zusätze sind zum Beispiel Tartrat- und Citrat-Puffer, Ethanol, Komplexbildner (wie Ethylendiamintetraessigsäure und deren nicht-toxische Salze). Zur Regelung der Viskosität kommen hochmolekulare Polymere in

Frage wie beispielsweise flüssiges Polyethylenoxid, mikrokristalline Cellulosen Carboxymethylcellulosen, Polyvinylpyrrolidone, Dextrane oder Gelatine. Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere wie Polyethylenglykol.

Ölige Suspensionen für parenterale oder topische Anwendungen können vegetabile synthetische oder semisynthetische Öle wie beispielsweise flüssige Fettsäureester mit jeweils 8 bis 22 C-Atomen in den Fettsäureketten, zum Beispiel Palmitin- , Laurin-, Tridecyl-, Margarin-, Stearin-, Arachin-, Myristin-, Behen-, Pentadecyl-, Linol-, Elaidin-, Brasidin-, Eruca- oder Ölsäure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sind, sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfett, Kokosfettsäure-isopropylester, Ölsäureoleylester,Ölsäuredecylester,Milchsäureethylester,Dibutylphthalat, Adipinsäurediisopropylester, Polyol-Fettsäureester u. a. Ebenso geeignet sind Silikonöle verschiedener Viskosität oder Fettalkohole wie Isotridecylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnussöl oder Sojabohnenöl Verwendung finden.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglykol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuran, Cyclohexanon etc.

Als Filmbildner können Celluloseether verwendet werden, die sich sowohl in Wasser als auch in organischen Lösungsmitteln lösen bzw. anquellen können, wie beispielsweise Hydroxypropylmethylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie z. B. Natriumcarboxymethylcellulose, Polyacrylsäure, Polymethacrylsäure und deren Salze, Natriumamylopektinsemiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure. Auch die Verwendung von Tensiden, Emulgatoren oder Netzmitteln kann zur Formulierung notwendig sein, wie z. B. von Na-Laurylsulfat, Fettalkoholethersulfaten, Di-Na-N-lauryl-β-iminodipropionat, polyoxyethyliertes Rizinusöl oder Sorbitan-Monooleat, Sorbitan-Monostearat, Polysorbaten (z. B. Tween), Cetylalkohol, Lecithin, Glycerinmonostearat, Polyoxyethylenstearat, Alkylphenolpolyglykolether, Cetyltrimethylammoniumchlorid oder Mono-/Dialkylpolyglykolether-orthophosphorsäure-monoethanolaminsalzen. Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zubereitungen zur parenteralen Applikation können in separaten Dosiseinheitsformen wie z. B. Ampullen oder Vials vorliegen. Vorzugsweise werden Lösungen des Wirkstoffes verwendet, bevorzugt wässrige Lösungen und vor allem isotonische Lösungen aber auch Suspensionen. Diese Injektionsformen können als Fertigpräparat zur Verfügung gestellt werden oder erst direkt vor der Anwendung durch Mischen der wirksamen Verbindung, zum Beispiel des Lyophilisats, gegebenenfalls mit weiteren festen Trägerstoffen, mit dem gewünschten Lösungs- oder Suspensionsmittel zubereitet werden.

Intranasale Zubereitungen können als wässrige oder ölige Lösungen bzw. als wässrige oder ölige Suspensionen vorliegen. Sie können auch als Lyophilisate vorliegen, die vor der Anwendung mit dem geeigneten Lösungs- oder Suspensionsmittel zubereitet werden.

Die Herstellung, Abfüllung und Verschließung der Präparate erfolgt unter den üblichen antimikrobiellen und aspetischen Bedingungen.

Die Erfindung betrifft weiterhin Verfahren zur Herstellung der erfindungsgemäßen Verbindungen.

Erfindungsgemäß werden die Verbindungen der allgemeinen Formel 1 mit den zuvor dargestellten Bedeutungen von $R^1$, $R^2$, $R^3$ und n = 1 hergestellt,

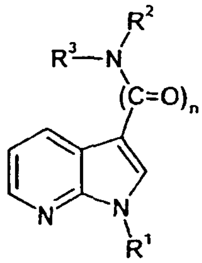

1

indem 7-Azaindol-3-carbonsäuren der Formel 2 mit identischer Bedeutung von $R^1$

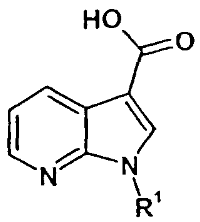

2

in an sich bekannter Weise mittels Säurechloriden, vorzugsweise mit Thionylchlorid oder Oxalylchlorid, zunächst in die analogen 7-Azaindol-3-carbonsäurechloride der Formel 3 überführt werden.

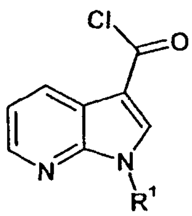

3

Aus den isolierten 7-Azaindol-3-carbonsäurechloriden der Formel 3 entstehen nachfolgend durch Umsetzung mit einem primären oder sekundären Amin die erfindungsgemäßen Verbindungen der allgemeinen Formel 1, mit den zuvor dargestellten Bedeutungen von $R^1$, $R^2$, $R^3$ und n = 1. Die Reaktion verläuft vorteilhaft in Gegenwart einer Hilfsbase. Als Hilfsbasen können ein Überschuss des als Reaktionspartner verwendeten Amins, ein tertiäres Amin, vorzugsweise Pyridin oder Triethylamin, sowie anorganische Basen, vorzugsweise Alkalihydroxide oder Alkalihydride verwendet werden.

Erfindungsgemäß werden die Verbindungen der allgemeinen Formel 1 mit den zuvor dargestellten Bedeutungen von $R^1$, $R^2$, $R^3$ und n = 2 hergestellt,

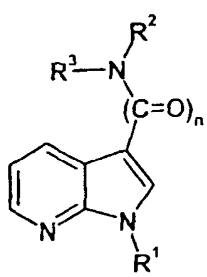

1

indem 7-Azaindole der Formel 4 mit identischer Bedeutung von $R^1$

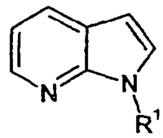

4

in an sich bekannter Weise durch Acylierung mit Oxalylchlorid zunächst in die analogen 7-Azaindol-3-yl-glyoxylsäurechloride der Formel 5 überführt werden.

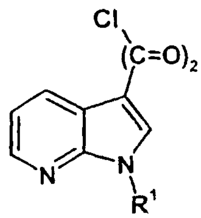

5

Aus den isolierten 7-Azaindol-3-yl-glyoxylsäurechloriden der Formel 5 entstehen nachfolgend durch Umsetzung mit einem primären oder sekundären Amin die erfindungsgemäßen Verbindungen der allgemeinen Formel 1 mit den zuvor dargestellten Bedeutungen von $R^1$, $R^2$, $R^3$ und n = 2. Die Reaktion verläuft vorteilhaft in Gegenwart einer Hilfsbase. Als Hilfsbasen können ein Überschuss des als Reaktionspartner verwendeten Amins, ein tertiäres Amin, vorzugsweise Pyridin oder Triethylamin, sowie anorganische Basen, vorzugsweise Alkalihydroxide oder Alkalihydride verwendet werden.

Ausführungsbeispiele

Exemplarisches Herstellungsverfahren für erfindungsgemäße Verbindungen der Formel 1 mit n = 1:

Beispiel 1: N-(4-Pyridylmethyl)-1-cyclopropylmethyl-7-azaindol-3-carbonsäureamid

1,87 g 1-Cyclopropylmethyl-7-azaindol-3-carbonsäure (8,6 mmol) werden in 15 ml Dichlormethan suspendiert. Unter Kühlung mit Wasser werden 1,8 ml Oxalylchlorid (17,4 mmol) zugegeben. Das Reaktionsgemisch wird 8 Stundengerührt. Dabei kristallisiertdas 11-Cyclopropylmethyl-7-azaindol-3-carbonsäurechlorid aus. Es wird isoliert und in 18 ml Tetrahydrofuran (THF) gelöst.

1,14 g Natriumhydrid (60 %ig) werden in 21 ml THF suspendiert. Unter Rühren bei ca. 10 ° C wird eine Lösung von 0,93 g 4-Aminomethylpyridin (8,6 mmol) in 21 ml THF zugetropft. Nach ca. 15 Minuten wird die zuvor hergestellte Lösung des 1-Cyclopropylmethyl-7-azaindol-3-carbonsäurechlorid zum Reaktionsgemisch zugetropft. Danach wird das Ganze 3 Stunden am Rückfluss gekocht. Nach Abkühlung wird das Reaktionsgemisch mit 36 ml Essigsäureethylester und 36 ml Wasser versetzt. Die Phasen werden getrennt und die organische Phase mit Wasser gewaschen. Das Lösungsmittel wird abdestilliert und der Rückstand aus Ethanol umkristallisiert.
Ausbeute: 1,3 g (50 % d. Theorie)
Schmelzpunkt: 187 - 189 °C

Unter Verwendung des angegebenen Herstellungsverfahrens können zahlreiche weitere Verbindungen der Formel 1 mit n = 1 hergestellt werden, von denen folgende beispielhaft angeführt werden:

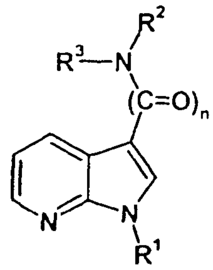

1

| Beispiel | $R^1$ | $-NR^2R^3$ | n | Schmelzpkt. [°C] |
|---|---|---|---|---|
| 1 | Cyclopropylmethyl- | 4-Pyridylmethylamino- | 1 | 187-189 Ethanol |
| 2 | Isobutyl- | 3,5-Dichlor-4-pyridylamino- | 1 | 168-170 Ethanol |
| 3 | n-Hexyl- | 3,5-Dichlor-4-pyridylamino- | 1 | 136-137 Methanol |
| 4 | Cyclopropylmethyl- | 3,5-Dichlor-4-pyridylamino | 1 | 186-187 Ethanol |
| 5 | 4-Fluorbenzyl- | 4-Pyridylmethyl-amino- | 1 | 189-191 Ethanol |
| 6 | 4-Fluorbenzyl- | 3, 5-Dichlor-4-pyridylamino- | 1 | 232-233 Ethanol |
| 7 | 4-Methoxy-benzyl | 3,5-Dichlor-4-pyridylamino- | 1 | 193-195 Ethanol |
| 8 | 4-Chlorbenzyl- | 4-Pyridylamino- | 1 | 192-194 Methanol |
| 9 | 4-Fluorbenzyl- | Morpholino- | 1 | 182-184 Ethanol |
| 10 | 2-Methylpropen-3-yl- | 2,6-Dichlorphenylamino- | 1 | 171-174 Ethanol |
| 11 | 4-Pyridylmethyl- | 3, 5-Dichlor-4-pyridylamino | 1 | 190-192 Methanol |

Exemplarisches Herstellungsverfahren für erfindungsgemäße Verbindungen der Formel 1 mit n = 2:

Beispiel 12

N-(3,5-Dichlorpyridin-4-yl)-[1-(3-methoxybenzyl)-7-azaindol-3-yl]-gyoxylsäureamid

3,57 g 1-(3-Methoxybenzyl)-7-azaindol (15 mmol) werden in 50 ml tert. Butylmethylether gelöst. Bei 0 °C wird unter Rühren eine Lösung von 1, 54 ml Oxalylchlorid (18 mmol) in 10 ml tert. Butylmethylether zugetropft. Danach wird das Gemisch 2 Stunden am Rückfluss gekocht. Anschließend wird das Lösungsmittel im Vakuum abdestilliert. Das entstandene 1-(3-Methoxybenzyl)-7-azaindol-3-yl-glyoxylsäurechlorid wird als fester Rückstand erhalten, der in 50 ml Tetrahydrofuran (THF) suspendiert wird.

Zu einer Suspension von 2 g Natriumhydrid in 20 ml THF wird bei -5 °C eine Lösung von 2,4 g 4-Amino-3,5-dichlorpyridin (15 mmol) in 30 ml THF zugetropft. Unter Rühren wird das Gemisch danach 1 Stunde lang auf 20 °C temperiert. Anschließend wird die zuvor hergestellte Suspension des 1-(3-Methoxybenzyl)-7-azaindol-3-yl-glyoxylsäurechlorids bei ca. 0 °C zugetropft. Schließlich wird die Reaktionsmischung 4 Stunden am Rückfluss gekocht. Das

Lösungsmittel wird im Vakuum entfernt. Der Rückstand wird mit 50 ml Essigsäureethylester und 50 ml Wasser verrührt. Die Phasen werden getrennt. Die organische Phase wird mit Wasser gewaschen. Das Lösungsmittel wird im Vakuum abdestilliert. Der Rückstand wird aus Isopropanol umkristallisiert.
Ausbeute: 3,5 g (51,5 % d. Theorie)
Schmelzpunkt: 165 - 167 °C

Unter Verwendung des angegebenen Herstellungsverfahrens können zahlreiche weitere Verbindungen der Formel 1 mit n = 2 hergestellt werden, von denen folgende beispielhaft angeführt werden:

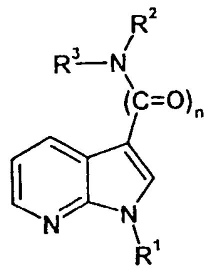

1

| Beispiel | $R^1$ | $-NR^2R^3$ | n | Schmelz-pkt. [°C] |
|---|---|---|---|---|
| 12 | 3-Methoxybenzyl- | 3,5-Dichlor-4-pyridylamino- | 2 | 165-167 Isopropanol |
| 13 | 4-Fluorbenzyl- | 4-Pyridylamino- x HCl | 2 | 275-278 zers. DMF |
| 14 | 4-Fluorbenzyl- | 3,5-Dichlor-4-pyridylamino | 2 | 201-202 Ethanol |
| 15 | 4-Chlorbenzyl- | 4-Pyridylamino- x HCl | 2 | 280-283 zers. DMF |
| 16 | 4-Chlorbenzyl- | 3,5-Dichlor-4-pyridylamino | 2 | 205-207 Ethanol |
| 17 | 4-Methoxybenzyl- | 3,5-Dichlor-4-pyridylamino | 2 | 165-167 Ethanol |
| 18 | 4-Chlorbenzyl | 2,6-Dichlorphenyl-amino- | 2 | 166-168 Ethanol |
| 19 | 4-Fluorbenzyl- | 4-Carboxy-phenylamino | 2 | 279-282 Isopro-panol |

| | | | | |
|---|---|---|---|---|
| 20 | 4-Fluorbenzyl- | 4-Ethoxy-carbonylphenyl-amino- | 2 | 209-211 Ethanol |
| 21 | 4-Fluorbenzyl- | 3,4-Dimethoxy-phenylamino- | 2 | 173-176 Ethanol |
| 22 | 4-Methylbenzyl- | 3,5-Dichlor-4-pyridylamino- | 2 | 176-178 Ethanol |
| 23 | 4-Hydroxybenzyl- | 3,5-Dichlor-4-pyridylamino- | 2 | 140-142 Ethanol |
| 24 | 3-Hydroxybenzyl- | 3,5-Dichlor-4-pyridylamino- | 2 | 241-244 Ethanol |
| 25 | Cyclopropyl-methyl- | 3,5-Dichlor-4-pyridylamino- | 2 | 215-218 Ethanol |
| 26 | n-Hexyl- | 3,5-Dichlor-4-pyridylamino- | 2 | 165-167 Ethanol |
| 27 | Isobutyl- | 3,5-Dichlor-4-pyridylamino- | 2 | 152-154 Methanol |
| 28 | 2-Methyl-propen-3-yl- | 3,5-Dichlor-4-pyridylamino- | 2 | 114-116 Methanol |
| 29 | 2-Methoxyethyl- | 3,5-Dichlor-4-pyridylamino- | 2 | 166-168 Methanol |
| 30 | 1-Naphthylmethyl- | 3,5-Dichlor-4-pyridylamino- | 2 | 181-183 Ethanol |
| 31 | 4-Pyridylmethyl- | 3,5-Dichlor-4-pyridylamino- | 2 | 199-201 Ethanol |
| 32 | 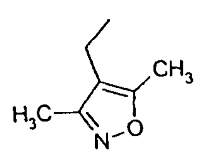 | 3,5-Dichlor-4-pyridylamino- | 2 | 196-198 Ethanol |
| 33 | 4-Fluorbenzyl- | $-N(C_2H_4-OCH_3(_2$ | 2 | 63-66 Methanol |
| 34 | 4-Fluorbenzyl- | 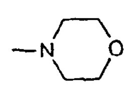 | 2 | 184-185 Ethanol |

| | | | | |
|---|---|---|---|---|
| 35 | 4-Fluorbenzyl- | 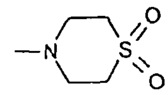 | 2 | 188-191 Ethanol |
| 36 | 4-Fluorbenzyl- | 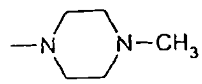 | 2 | 179-181 Methanol |
| 37 | 4-Fluorbenzyl- | 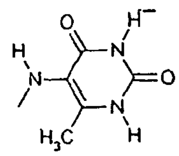 | 2 | 297-300 zers. DMF |
| 38 | 4-Fluorbenzyl- | 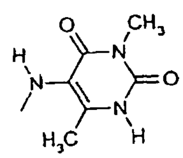 | 2 | 310-313 DMF |
| 39 | 4-Fluorbenzyl- | 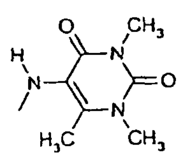 | 2 | 160-162 Aceton |
| 40 | 4-Fluorbenzyl- | 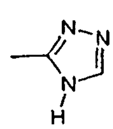 | 2 | 312-315 zers. DMF |

Die erfindungsgemäßen Verbindungen sind starke Inhibitoren der Phospho-diesterase 4 und der TNFα Freisetzung. Ihr therapeutisches Potential wird in vivo beispielsweise durch die Hemmung der asthmatischen Spätphase-Reaktion (Eosinophilie) sowie durch die Beeinflussung der Allergen-induzierten vaskulären Permeabilität an aktiv sensibilisierten Brown-Norway Ratten belegt.

Inhibition der Phosphodiesterase

Die PDE 4-Aktivität wird in Enzympräparationen aus humanen polymorphkernigen Lymphocyten (PMNL) bestimmt, die PDE 2, 3 und 5-Aktivität mit PDE aus humanen Thrombocyten. Humanes Blut wurde mit Citrat anticoaguliert. Durch eine Zentrifugation bei 700 x g für 20 Minuten bei RT wird das thrombocytenreiche Plasma im Überstand von den Erythrocyten und Leukocyten getrennt. Die Thrombocyten werden durch Ultraschall lysiert und im PDE 3 und PDE 5-Assay eingesetzt. Für die Bestimmung der PDE 2-Aktivität wird die cytosolische Thrombocytenfraktion über einer Anionenaustauschersäule mittels NaCl-Gradienten gereinigt und der PDE 2-Peak wird für den Assay gewonnen. Die PMNLs für die PDE 4-Bestimmung werden durch eine folgende Dextransedimentation und anschließende Gradientenzentrifugation mit Ficoll-Paque isoliert. Nach einem zweimaligen Waschen der Zellen werden die noch enthaltenden Erythrocyten durch die Zugabe von 10 ml hypotonischem Puffer (155 mM NH4Cl, 10 mM NaHCO3, 0,1 mM EDTA, pH=7,4) innerhalb von 6 Minuten bei 4 °C lysiert. Die noch intakten PMNLs werden noch zwei Mal mit PBS gewaschen und mittels Ultraschall lysiert. Der Überstand einer einstündigen Zentrifugation bei 4 ° C bei 48000 × g enthält die cytosolische Fraktion der PDE 4 und wird für die PDE 4-Messungen eingesetzt.

Die Phosphodiesterase-Aktivität wird mit einigen Modifizierungen nach der von Thompson et al. beschriebenen Methode bestimmt. (Thompson, W.J.; Appleman, M.M., Assay of cyclic nucleotide phosphodiesterase and resolution of multiple molecular forms of the enzyme. Adv. Cycl. Nucl. Res. 1979, 10, 69-92).

Die Reaktionsmischungen enthalten 50 mM Tris-HCl (pH 7,4), 5 mM $MgCl_2$, die Inhibitoren in variablen Konzentrationen, die entsprechende Enzympräparation sowie die zur Erfassung der einzelnen Isoenzyme notwendigen weiteren Komponenten (siehe unten). Durch die Zugabe des Substrates 0,5 µM [$^3$H]-cAMP oder [$^3$H]-cGMP (ca. 6000 CPM/Test) wird die Reaktion gestartet. Das Endvolumen beträgt 100 ml. Testsubstanzen werden als Stammlösungen in DMSO angesetzt. Die DMSO-Konzentration im Reaktionsgemisch ist 1 % v/v. Bei dieser DMSO-Konzentration wird die PDE-Aktivität nicht beeinflusst. Nach dem Start der Reaktion mittels Substrat-Zugabe werden die Proben 30 Minuten bei 37°C inkubiert. Durch ein Erhitzen der Testtubes für 2 Minuten auf 110 °C wird die Reaktion gestoppt. Die Proben bleiben für weitere 10 Minuten im Eis. Nach der Zugabe von 30 µl 5 '-Nukleotidase (1 mg/ml, aus einer Schlangengiftsuspension aus Crotalus adamanteus) erfolgt eine Inkubation für 10 Minuten bei 37 °C. Die Proben werden auf Eis abgestoppt, jeweils 400 µl einer Mischung aus Dowex-Wasser-Ethanol (1+1+1) zugegeben, gut gemixt und wieder 15 Minuten auf Eis inkubiert. Die Reaktionsgefäße werden 20 Minuten bei 3000 x g zentrifugiert. 200 µl Aliquotes des Überstandes werden direkt in Szintillationgefäße überführt. Nach der Zugabe von 3 ml Szintillator werden die Proben im Betacounter gemessen.

Für die Bestimmung der PDE 4, 3 und 2-Aktivität wird als Substrat [$^3$H]-cAMP, für die Bestimmung der PDE 5-Aktivität [$^3$H]-cGMP verwendet. Die jeweils unspezifischen Enzymaktivitäten werden in Gegenwart von 100 µM Rolipram bei der PDE 4 und in Gegenwart von 100 µM IBMX bei der Bestimmung der PDE 3 und 5 ermittelt und von den Testwerten subtrahiezt. Die Inkubationsansätze des PDE 3-Assays enthalten 10 µM Rolipram, um eventuelle Verunreinigungen durch die PDE 4 zu hemmen. Die PDE 2 wird mit einem SPA-Assay der Firma Amersham getestet. In Gegenwart des Aktivators der PDE 2 (5 µM cGMP) wird der Assay durchgeführt.

Für die erfindungsgemäßen Verbindungen wurden bezüglich der Inhibition der Phosphodiesterase 4 $IC_{50}$ -Werte im Bereich von $10^{-9}$ bis $10^{-5}$ M bestimmt. Die Selektivität gegenüber den PDE - Typen 2, 3 und 5 beträgt Faktor 100 bis 10.000.

Exemplarisch wurden für ausgewählte Anwendungsbeipiele die Ergebnisse zur Hemmung der PDE 4 in nachfolgender Tabelle zusammengestellt:

| Beispiel | Hemmung der PDE 4 $IC_{50}$ [µmol/l] |
|---|---|
| 1 | 0.710 |
| 2 | 1.400 |
| 12 | 0.005 |
| 13 | 0.058 |
| 14 | 0.004 |
| 15 | 0.031 |
| 16 | 0.002 |

(fortgesetzt)

| Beispiel | Hemmung der PDE 4 $IC_{50}$ [µmol/l] |
|---|---|
| 17 | 0.008 |
| 18 | 0.031 |
| 22 | 0.002 |
| 23 | 0.001 |
| 24 | 0.003 |
| 25 | 0.004 |
| 26 | 0.021 |
| 27 | 0.002 |
| 28 | 0.003 |
| 32 | 0.113 |
| 37 | 0.987 |

Hemmung der TNFα Freisetzung aus Zellen nasaler Polypen

Die Versuchsanordnung entspricht im Wesentlichen der von Campbell, A.M. und Bousquet J (Anti-allergic activity of $H_1$-blockers. Int. Arch. Allergy Immunol., 1993, 101, 308-310) beschriebenen Methode. Das Ausgangsmaterial bilden nasale Polypen (OP-Material) von Patienten die sich einer chirurgischen Behandlung unterzogen haben.

Das Gewebe wird mit RPMI 1640 gewaschen und anschließend mit Protease (2.0 mg/ml), Collagenase (1.5 mg/ml), Hyaluronidase (0.75 mg/ml) und DNAse (0.05 mg/ml) über 2 h bei 37 °C aufgeschlossen (1 g Gewebe auf 4 ml RPMI 1640 mit Enzymen). Die erhaltenen Zellen, eine Mischung aus Epithelzellen, Monozyten, Makrophagen, Lymphozyten, Fibroblasten und Granulozyten, werden filtriert und durch wiederholtes Zentrifugieren in Nährlösung gewaschen, durch Zugabe von humanem IgE passiv sensibilisiert und die Zellsuspension auf eine Konzentration von 2 Mio Zellen/ml in RPMI 1640 (ergänzt mit Antibiotika, 10 % fetalem Kälberserum, 2 mM Glutamin und 25 mM Hepes) eingestellt. Diese Suspension wird auf 6-Well-Zellkulturplatten (1 ml/Well) verteilt. Die Zellen werden mit den Prüfsubstanzen in verschiedenen Endkonzentrationen 30 min vorinkubiert und anschließend durch Zugabe von Anti-IgE (7,2 µg/ml) zur TNFα Freisetzung angeregt. Die maximale Freisetzung in das Nährmedium erfolgt nach ca. 18 Stunden. In diesem Zeitraum werden die Zellen bei 37 °C und 5 % $CO_2$ inkubiert. Das Nährmedium (Überstand) wird durch Zentrifugation gewonnen (5 min 4000 U/min) und bis zur Zytokinbestimmung bei -70 °C gelagert. Die Bestimmung von TNFα im Überstand erfolgt mit sog. Sandwich-ELISAs (Grundmaterial Pharmingen), mit denen Konzentrationen des Zytokins im Bereich von 30-1000 pg/ml nachgewiesen werden können.

Nicht mit Anti-IgE stimulierte Zellen produzieren kaum TNFα, stimulierte Zellen dagegen sezernieren große Mengen an TNFα, was z.B. durch PDE4 Inhibitoren dosisabhängig vermindert werden kann. Aus der prozentualen Hemmung (TNFα-Freisetzung der mit Anti-IgE stimulierte Zellen = 100 %) der geprüften Substanzen bei verschiedenen Konzentrationen wird die $IC_{50}$ (concentration at 50 % inhibition) berechnet.

Für die erfindungsgemäßen Verbindungen wurden $IC_{50}$ - Werte im Bereich von $10^{-7}$ bis $10^{-5}$ M bestimmt.

Exemplarisch wurden für ausgewählte Anwendungsbeipiele die Ergebnisse zur Hemmung der TNFα Freisetzung in nachfolgender Tabelle zusammengestellt:

| Beispiel | Hemmung der TNFα Freisetzung | |
|---|---|---|
| | Konzentration | Hemmung |
| 14 | 0,3 µmol/l | 92 |
| 16 | 1,0 µmol/l | 90 |
| 17 | 1,0 µmol/l | 91 |
| 27 | 1,0 µmol/l | 91 |

Hemmung der Spätphasen-Eosinophilie 48 h nach inhalativer Ovalbuminchallenge an aktiv sensibilisierten Brown Norway Ratten

Die Hemmung der pulmonalen Eosinophilen-Infiltration durch die erfindungsgemäßen Substanzen wird an aktiv gegen Ovalbumin (OVA) sensibilisierten männlichen Brown Norway Ratten (200-250 g) geprüft. Die Sensibilisierung erfolgt durch subcutane Injektionen einer Suspension aus 10 µg OVA zusammen mit 20 mg Aluminiumhydroxid als Adjuvans in 0,5 ml physiologischer Kochsalzlösung pro Tier am Tag 1, 14 und 21. Zusätzlich dazu erhalten die Tiere zu den gleichen Zeitpunkten *Bordetella pertussis* vaccine Verdünnung pro Tier 0,25 ml i.p. gespritzt. Am 28. Versuchstag werden die Tiere einzeln in offene 1 l Plexiglasboxen gesetzt, die an ein Kopf-Nasen Expositionsgerät angeschlossen sind. Die Tiere werden einem Aerosol aus 1,0 %iger Ovalbuminsuspension ausgesetzt (Allergen-Challenge). Das Ovalbumin-Aerosol wird durch einen mit Druckluft (0,2 MPa) betriebenen Vernebler (Bird micro nebulizer, Palm Springs CA, USA) erzeugt. Die Expositionszeit beträgt 1 Stunde, wobei Normalkontrollen mit einem Aerosol aus 0,9%iger Kochsalzlösung ebenfalls 1 Stunde lang vernebelt werden.

48 Stunden nach der Allergen-Challenge kommt es zu einer massiven Einwanderung von eosinophilen Granulozyten in die Lungen der Tiere. Zu diesem Zeitpunkt werden die Tiere mit einer Überdosis Ethylurethan (1,5 g/kg Körpergewicht i.p.) narkotisiert und eine bronchoalveoläre Lavage (BAL) mit 3 × 4 ml Hank's Balance-Lösung durchgeführt. Die Gesamtzellzahl und die Anzahl der eosinophilen Granulozyten der gepoolten BAL-Flüssigkeit werden anschließend mit einem automatischen Zelldifferenzierungsgerät (Bayer Diagnostics Technicon H 1 E) bestimmt. Für jedes Tier werden die Eosinophilen (EOS) in der BAL in Mio/Tier berechnet: EOS/µl × BAL-Recovery (ml) = EOS/Tier.

Bei jedem Test werden 2 Kontrollgruppen (Vernebelung mit physiologischer Kochsalzlösung und Vernebelung mit OVA-Lösung) mitgeführt.

Die prozentuale Hemmung der Eosinophilie der mit Substanz behandelten Versuchsgruppe wird nach folgender Formel berechnet:

$$\{((OVAC - SC) - (OVAD - SC)) / (OVAC - SC)\} \times 100\ \% = \%\ Hemmung$$

(SC = Vehicel behandelte und mit 0,9 %iger Kochsalzlösung gechallengte Kontrollgruppe; OVAC = Vehicel behandelte und mit 1 %iger Ovalbuminsuspension gechallengte Kontrollgruppe; OVAD = Substanz behandelte und mit 1 %iger Ovalbuminsuspension gechallengte Versuchsgruppe)

Die Testsubstanzen werden intraperitoneal oder oral als Suspension in 10 % Polyethylenglycol 300 und 0,5 %iger 5-Hydroxyethylcellulose 2 Stunden vor der Allergen-Challenge appliziert. Die Kontrollgruppen werden entsprechend der Applikationsform der Testsubstanz mit dem Vehicel behandelt.

Die erfindungsgemäßen Verbindungen hemmen die Spätphasen-Eosinophilie nach intraperitonealer Applikation von 10 mg/kg um 30 % bis 100 % und nach oraler Applikation von 30 mg/kg um 30 % bis 75 %.

Die erfindungsgemäßen Verbindungen sind somit besonders geeignet für die Herstellung von Arzneimitteln zur Behandlung von Erkrankungen, die mit dem Wirken von Eosinophilen verbunden sind.

Exemplarisch wurden für ausgewählte Anwendungsbeipiele die Ergebnisse zur Hemmung der Eosinophilie in nachfolgender Tabelle zusammengestellt:

| Beispiel | Hemmung der Eosinophilie | |
|---|---|---|
| | Dosis/Applikation | Hemmung [%] |
| 14 | 10 mg/kg i.p. | 62 |
| | 10 mg/kg p.o. | 59 |
| 16 | 10 mg/kg i.p. | 100 |
| | 10 mg/kg p.o. | 70 |
| 17 | 10 mg/kg i.p. | 75 |
| | 10 mg/kg p.o. | 32 |
| 27 | 10 mg/kg i.p. | 50 |
| | 10 mg/kg p.o. | 70 |

Hemmung der Lipopolysaccharid (LPS)-induzierten Lungen-Neutrophilie in Lewis Ratten

Die Hemmung der pulmonalen Neutrophilen-Infiltration durch die erfindungsgemäßen Substanzen wird an männlichen Lewis Ratten (250-350 g) geprüft. Am Versuchstag werden die Tiere einzeln in offene 1 l Plexiglasboxen gesetzt, die an ein Kopf-Nasen Expositionsgerät angeschlossen sind. Die Tiere werden einem Aerosol aus einer Lipopolysaccharidsuspension (100µg LPS/ml 0,1 % Hydroxylamin-Lösung) in PBS ausgesetzt (LPS-Provokation). Das LPS/Hydroxylamin-Aerosol wird durch einen mit Druckluft (0,2 MPa) betriebenen Vernebler (Bird micro nebulizer, Palm Springs CA, USA) erzeugt. Die Expositionszeit beträgt 40 Minuten, wobei Normalkontrollen mit einem Aerosol aus 0,1 %iger Hydroxylamin-Lösung in PBS ebenfalls 40 Minuten lang vernebelt werden.

6 Stunden nach der LPS-Provokation kommt es zu einer maximalen, massiven Einwanderung von neutrophilen Granulozyten in die Lungen der Tiere. Zu diesem Zeitpunkt werden die Tiere mit einer Überdosis Ethylurethan (1,5 g/kg Körpergewicht i.p.) narkotisiert und eine bronchoalveoläre Lavage (BAL) mit 3 × 4 ml Hank's Balance-Lösung durchgeführt. Die Gesamtzellzahl und die Anzahl der neutrophilen Granulozyten der gepoolten BAL-Flüssigkeit werden anschließend mit einem automatischen Zelldifferenzierungsgerät (Bayer DiagnosticsTechnicon H 1 E) bestimmt. Für jedes Tier werden die Neutrophilen (NEUTRO) in der BAL in Mio/Tier berechnet: NEUTRO/µl × BAL-Recovery (ml) = NEUTRO/Tier.

Bei jedem Test werden 2 Kontrollgruppen (Vernebelung mit 0,1%iger Hydroxylamin-Lösung in PBS und Vernebelung mit 100 µg LPS/ml 0,1 % Hydroxylamin-Lösung in PBS) mitgeführt.

Die prozentuale Hemmung der Neutrophilie der mit Substanz behandelten Versuchsgruppe wird nach folgender Formel berechnet:

$$\{((LPSC - SC) - (LPSD - SC)) / (LPSC - SC)\} \times 100\ \% = \%\ Hemmung$$

SC = Vehicel behandelte und mit 0,1 %iger Hydroxylamin-Lösung gechallengte Kontrollgruppe; LPSC = Vehicel behandelte und mit LPS (100 µg/ml 0,1 %iger Hydroxylamin-Lösung) gechallengter Kontrollgruppe; LPSD = Substanz behandelte und mit LPS (100 µg/ml 0,1 %iger Hydroxylamin-Lösung) gechallengter Versuchsgruppe.

Die Testsubstanzen werden oral als Suspension in 10 % Polyethylenglycol 300 und 0,5 %iger 5-Hydroxyethylcellulose 2 Stunden vor der LPS-Provokation appliziert. Die Kontrollgruppen werden entsprechend der Applikationsform der Testsubstanz mit dem Vehicel behandelt.

Die erfindungsgemäßen Verbindungen hemmen die Neutrophilie nach oraler Applikation von 1 mg/kg um 40% bis 90 % und sind somit besonders geeignet für die Herstellung von Arzneimitteln zur Behandlung von Erkrankungen, die mit dem Wirken von Neutrophilen verbunden sind.

Exemplarisch wurden für ausgewählte Anwendungsbeipiele die Ergebnisse zur Hemmung der Neutrophilie in nachfolgender Tabelle zusammengestellt:

| Beispiel | Hemmung der Neutrophilie | |
|---|---|---|
| | Dosis/Applikation | Hemmung [%] |
| 14 | 1 mg/kg p.o | 80 |
| 22 | 1 mg/kg p.o. | 64 |
| 27 | 1 mg/kg p.o. | 52 |

**Patentansprüche**

1. 7-Azaindole der Formel 1

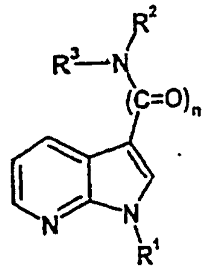

1

worin

n = 1 oder 2 sein kann, und

$R^1$ für

-$C_{1...10}$-Alkyl, geradkettig oder verzweigtkettig, unsubstituiert oder ein- oder mehrfach substituiert mit -OH, -SH, -$NH_2$, -$NHC_{1...6}$-Alkyl, -N($C_{1...6}$-Alkyl)$_2$, -$NHC_{6...14}$Aryl, -N($C_{6...14}$Aryl)$_2$, -N($C_{1...6}$Alkyl)($C_{6...14}$Aryl), -$NO_2$, -CN, -F, -Cl, -Br, -I, -O-$C_{1...6}$-Alkyl, -O-$C_{6...14}$-Aryl, -S-$C_{1...6}$-Alkyl, -S-$C_{6...14}$Aryl, -$SO_3H$, -$SO_2C_{1...6}$Alkyl, -$SO_2C_{6...14}$Aryl, -$OSO_2C_{1...6}$Alkyl, -$OSO_2C_{6...14}$Aryl, -COOH, -(CO)$C_{1...5}$Alkyl, mit mono-, bi- oder tricyclischen gesättigten oder ein- oder mehrfach ungesättigten Carbocyclen mit 3...14 Ringgliedern, mit mono-, bi- oder tricyclischen gesättigten oder ein- oder mehrfach ungesättigten Heterocyclen mit 5...15 Ringgliedern und 1...6 Heteroatomen, die vorzugsweise N, O und S sind, wobei die $C_{6...14}$ Aryl-Gruppen und die carbocyclischen und heterocyclischen Substituenten ihrerseits unsubstituiert oder ein- oder mehrfach mit $R^4$ substituiert sein können,

-$C_{2...10}$-Alkenyl, ein oder mehrfach ungesättigt, geradkettig oder verzweigtkettig, unsubstituiert, oder ein- oder mehrfach substituiert mit -OH, -SH, -$NH_2$, -$NHC_{1...6}$-Alkyl, -N($C_{1...6}$-Alkyl)$_2$, -$NHC_{6...14}$Aryl, -N($C_{6...14}$Aryl)$_2$, -N ($C_{1...6}$Alkyl)($C_{6...14}$Aryl), -$NO_2$, -CN, -F, -Cl, -Br, -I, -O-$C_{1...6}$-Alkyl, -O-$C_{6...14}$-Aryl, -S-$C_{1...6}$-Alkyl, -S-$C_{6...14}$Aryl, -$SO_3H$, -$SO_2C_{1...6}$Alkyl, -$SO_2C_{6...14}$Aryl, -$OSO_2C_{1...6}$Alkyl, -$OSO_2C_{6...14}$Aryl, -COOH, -(CO)$C_{1...5}$Alkyl, mit mono-, bi- oder tricyclischen gesättigten oder ein- oder mehrfach ungesättigten Carbocyclen mit 3 ...14 Ringgliedern, mit mono-, bi- oder tricyclischen gesättigten oder ein- oder mehrfach ungesättigten Heterocyclen mit 5...15 Ringgliedern und 1...6 Heteroatomen, die vorzugsweise N, O und S sind, wobei die $C_{6...14}$Aryl-Gruppen und die carbocyclischen und heterocyclischen Substituenten ihrerseits unsubstituiert oder ein- oder mehrfach mit $R^4$ substituiert sein können,

steht,

$R^2$ und $R^3$ gleich oder verschieden sein können, wobei nur einer von beiden für Wasserstoff stehen kann und $R^2$ und $R^3$ weiterhin

-$C_{1...5}$-Alkyl, unsubstituiert oder

ein- oder mehrfach substituiert mit -OH, -SH, -$NH_2$, -$NHC_{1...6}$-Alkyl, -N($C_{1...6}$-Alkyl)$_2$, -$NO_2$, -CN, -F, -Cl, -Br, -I, -O-$C_{1...6}$-Alkyl, -S-$C_{1...6}$-Alkyl, -Phenyl, -Pyridyl

-Phenyl, unsubstituiert oder ein- oder mehrfach substituiert mit -OH, -SH, -$NH_2$, -$NHC_{1...3}$-Alkyl, -N($C_{1...3}$-Alkyl)$_2$, -$NO_2$, -CN, -COOH, -$COOC_{1...3}$Alkyl, -F, -Cl, -Br, -O-$C_{1...3}$-Alkyl, -S-$C_{1...3}$-Alkyl,

-Pyridyl, unsubstituiert oder ein- oder mehrfach substituiert mit -$NO_2$, -CN, -COOH, -$COOC_{1...3}$Alkyl, -Cl, -Br, -O-$C_{1...3}$-Alkyl, -S-$C_{1...3}$-Alkyl,

sowie

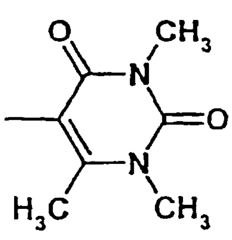

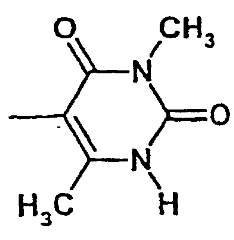

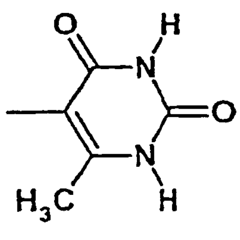

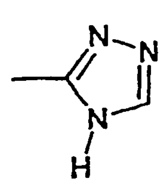

bedeuten können,

weiterhin die Gruppe -$NR^2R^3$ zusammen für

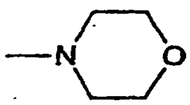 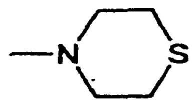 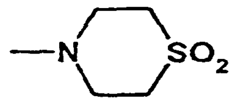 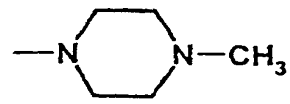

stehen kann,
und
$R^4$ für
-H, -OH, -SH, $-NH_2$, $-NHC_{1...6}$-Alkyl, $-N(C_{1...6}$-Alkyl$)_2$, , $-NHC_{6...14}$Aryl, $-N(C_{6...14}$Aryl$)_2$, $-N(C_{1...6}$Alkyl$)(C_{6...14}$Aryl), $-NHCOC_{1...6}$Alkyl, $-NO_2$, -CN, -COOH, $-COOC_{1...6}$Alkyl, $-(CO)C_{1...6}$Alkyl, $-(CS)C_{1...6}$Alkyl, -F, -Cl, -Br, -I, $-O-C_{1...6}$-Alkyl, $-O-C_{6...14}$-Aryl, $-S-C_{1...6}$-Alkyl, $-S-C_{6...14}$Aryl, $-SOC_{1...6}$Alkyl, $-SO_2C_{1...6}$Alkyl steht,
mit der Maßgabe, dass, wenn n = 1 nicht gleichzeitig
$R_1$ = $-C_{1...6}$-Alkyl,
$R_2$ = -H oder $-C_{1...6}$-Alkyl und
$R_3$ =

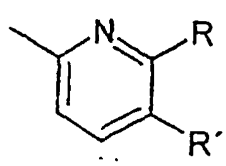 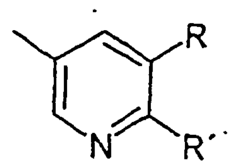 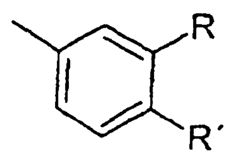

wobei R, R' unabhängig voneinander $-C_{1...6}$-Alkyl, gegebenenfalls ein- oder mehrfach mit Halogen substituiert, $-C_{2...6}$-Alkenyl, $-C_{3...6}$-Cycloalkyloxy, $-C_{3...6}$-Cycloalkyl$C_{1...6}$alkoxy, $-C_{2...6}$Alkinyl, $-C_{3...6}$Cycloalkyl, $-C_{3...6}$-Cycloalkyl$C_{1...6}$alkyl, $-C_{1...6}$Alkylthio, $-C_{3...6}$Cycloalkylthio, $-C_{3...6}$-Cycloalkyl$C_{1...6}$alkylthio, $-C_{1...6}$Alkoxy, Hydroxy-, Halogen-, Nitro-, $-CF_3$, $-C_2F_5$, $-OCF_3$, $-SCF_3$, $-SO_2CF_3$, $-SO_2F$, Formyl-, $C_{2...6}$Alkanoyl, Cyano-, gegebenenfalls substituiertes Phenyl- oder Thienyl-, -NR''$_2$, -CONR''$_2$, -COOR'' sind
oder
R + R' zusammen ein 5-gliedriger Carbozyklus oder Heterozyklus sind, und
R'' = -H oder $-C_{1...6}$Alkyl.

2. Physiologisch verträgliche Salze der Verbindungen nach Formel 1 gemäß Anspruch 1, **gekennzeichnet durch** Neutralisation der Basen mit anorganischen oder organischen Säuren bzw. **durch** Neutralisation der Säuren mit anorganischen oder organischen Basen bzw. **durch** Quaternierung tertiärer Amine zu quaternären Ammoniumsalzen.

3. Verbindungen nach Formel 1 gemäß Anspruch 1 und 2 mit einem asymmetrischen Kohlenstoffatom in der D-Form, der L-Form oder in Form von D,L-Mischungen, oder im Falle mehrerer asymmetrischer Kohlenstoffatome die diastereomeren Formen.

4. Verbindung nach Formel 1 gemäß einem der Anspruch 1 bis 3 mit n = 1, ausgewählt aus den folgenden Verbindungen:

N-(4-Pyridylmethyl)-1-cyclopropylmethyl-7-azaindol-3-carbonsäurea mid,
N-(3,5-Dichlorpyridin-4-yl)-1-isobutyl-7-azaindol-3-carbonsäureamid,
N-(3,5-Dichlorpyridin-4-yl)-1-hexyl-7-azaindol-3-carbonsäure-amid,
N-(3,5-Dichlorpyridin-4-yl)-1-cyclopropylmethyl-7-azaindol-3-carbonsäureamid,
N-(4-Pyridylmethyl)-1-(4-fluorbenzyl)-7-azaindol-3-carbonsäu-reamid,
N-(3,5-Dichlorpyridin-4-yl)-1-(4-fluorbenzyl)-7-azaindol-3-carbonsäureamid,
N-(3,5-Dichlorpyridin-4-yl)-1-(4-methoxybenzyl)-7-azaindol-3-carbonsäureamid,
N-(4-Pyridylmethyl)-1-(4-chlorbenzyl)-7-azaindol-3-carbon-säureamid,
1-(4-Fluorbenzyl)-7-azaindol-3-carbonsäuremorpholid,
N-(2, 6-Dichlorphenyl)-1-(2-methylpropen-3-yl)-7-azaindol-3-carbonsäureamid und
N-(3,5-Dichlorpyridin-4-yl)-1-(4-pyridylmethyl)-7-azaindol-3-carbonsäureamid.

**5.** Verbindung nach Formel 1 gemäß einem der Ansprüche 1 bis 3 mit n = 2, ausgewählt aus den folgenden Verbindungen:

N-(3,5-Dichlorpyridin-4-yl)-[1-(3-methoxybenzyl)-7-azaindol-3-yl]-glyoxylsäureamid,

N-(4-Pyridyl)-[1-(4-fluorbenzyl)-7-azaindol-3-yl]-glyoxylsäureamid-hydrochlorid,

N-(3,5-Dichlorpyridin-4-yl)-[1-(4-fluorbenzyl)-7-azaindol-3-yl]-glyoxylsäureamid,

N-(4-Pyridyl)-[1-(4-chlorbenzyl)-7-azaindol-3-yl]-glyoxylsäureamid-hydrochlorid,

N-(3,5-Dichlorpyridin-4-yl)-[1-(4-chlorbenzyl)-7-azaindol-3-yl]-glyoxylsäureamid,

N-(3, 5-Dichlorpyridin-4-yl)-[1-(4-methoxybenzyl)-7-azaindol-3-yl]-glyoxylsäureamid,

N-(2, 6-Dichlorphenyl)-[1-(4-chlorbenzyl)-7-azaindol-3-yl]-glyoxylsäureamid,

N-(4-Carboxyphenyl)-[1-(4-fluorbenzyl)-7-azaindol-3-yl]-glyoxylsäureamid,

N-(4-Ethoxycarbonylphenyl)-[1-(4-fluorbenzyl)-7-azaindol-3-yl]-glyoxylsäureamid,

N-(3,4-Dimethoxyphenyl)-[1-(4-fluorbenzyl)-7-azaindol-3-yl]-glyoxylsäureamid,

N-(3,5-Dichlorpyridin-4-yl)-[1-(4-methylbenzyl)-7-azaindol-3-yl]-glyoxylsäureamid,

N-(3,5-Dichlorpyridin-4-yl)-[1-(4-hydroxybenzyl)-7-azaindol-3-yl]-glyoxylsäureamid,

N-(3,5-Dichlorpyridin-4-yl)-[1-(3-hydroxybenzyl)-7-azaindol-3-yl]-glyoxylsäureamid,

N-(3,5-Dichlorpyridin-4-yl)-(1-cyclopropylmethyl-7-azaindol-3-yl)-glyoxylsäureamid,

N-(3,5-Dichlorpyridin-4-yl)-(1-hexyl-7-azaindol-3-yl)-glyoxylsäureamid,

N-(3,5-Dichlorpyridin-4-yl)-(1-isobutyl-7-azaindol-3-yl)-glyoxylsäureamid,

N-(3,5-Dichlorpyridin-4-yl)-[1-(2-methylpropen-3-yl)-7-azaindol-3-yl] -glyoxylsäureamid,

N-(3,5-Dichlorpyridin-4-yl)-[1-(2-methoxyethyl)-7-azaindol-3-yl]-glyoxylsäureamid,

N-(3, 5-Dichlorpyridin-4-yl)-[1-(1-naphthylmethyl)-7-azaindol-3-yl]-glyoxylsäureamid,

N-(3,5-Dichlorpyridin-4-yl)-[1-(4-pyridylmethyl)-7-azaindol-3-yl]-glyoxylsäureamid,

N-(3,5-Dichlorpyridin-4-yl)-[1-(3,5-dimethylisoxazol-4-ylmethyl)-7-azaindol-3-yl]-glyoxylsäureamid,

N,N-Bis(2-methoxyethyl)-[1-(4-fluorbenzyl)-7-azaindol-3-yl]-glyoxylsäureamid,

[1-(4-Fluorbenzyl)-7-azaindol-3-yl]-glyoxylsäuremorpholid,

[1-(4-Fluorbenzyl)-7-azaindol-3-yl]-glyoxylsäure-(S,S-dioxothiomorpholid),

[1-(4-Fluorbenzyl)-7-azaindol-3-yl]-glyoxylsäure (4-methylpiperazid),

N-(6-Methyluracil-5-yl)-[1-(4-fluorbenzyl)-7-azaindol-3-yl]-glyoxylsäureamid,

N-(3,6-Dimethyluracil-5-yl)-[1-(4-fluorbenzyl)-7-azaindol-3-yl]-glyoxylsäureamid,

N-(1,3,6-Trimethyluracil-5-yl)-[1-(4-fluorbenzyl)-7-azaindol-3-yl]-glyoxylsäureamid und

N-(1,2,4-4H-triazol-3-yl)-[1-(4-fluorbenzyl)-7-azaindol-3-yl]-glyoxylsäureamid.

6. Verfahren zur Herstellung von Verbindungen nach Formel 1 gemäß einem der Ansprüche 1 bis 4 mit n = 1, **gekennzeichnet dadurch, dass** 7-Azaindol-3-carbonsäuren mittels Säurechloriden in die analogen 7-Azaindol-3-carbonsäurechloride überführt und anschließend durch Reaktion mit primären oder sekundären Aminen zu den erfindungsgemäßen Verbindungen nach Formel 1 mit n = 1 umgesetzt werden.

7. Herstellung von Verbindungen nach Formel 1 nach dem Verfahren gemäß Anspruch 6, **gekennzeichnet durch** die Verwendung von Thionylchlorid oder Oxalylchlorid als Säurechloride zur Synthese der 7-Azaindol-3-carbonsäurechloride.

8. Herstellung von Verbindungen nach Formel 1 nach dem Verfahren gemäß Anspruch 6 oder 7, **gekennzeichnet durch** die Umsetzung der 7-Azaindol-3-carbonsäurechloride mit primären oder sekundären Aminen in Gegenwart einer Hilfsbase, vorzugsweise in Gegenwart eines Überschusses des als Reaktionspartner verwendeten Amins, eines tertiären Amins, beispielsweise von Pyridin oder Triethylamin, sowie anorganischer Basen, vorzugsweise Alkalihydroxide oder Alkalihydride.

9. Verfahren zur Herstellung von Verbindungen nach Formel 1 gemäß einem der Ansprüche 1 bis 3 und 5 mit n = 2, **gekennzeichnet dadurch, dass** 7-Azaindole mit Oxalylchlorid in die analogen 7-Azaindol-3-yl-glyoxylsäurechloride überführt und anschließend durch Reaktion mit primären oder sekundären Aminen zu den Verbindungen nach Formel 1 mit n = 2 umgesetzt werden.

10. Herstellung von Verbindungen nach Formel 1 nach dem Verfahren gemäß Anspruch 9, **gekennzeichnet durch** die Umsetzung der 7-Azaindol-3-yl-glyoxylsäurechloride mit primären oder sekundären Aminen in Gegenwart einer Hilfsbase, vorzugsweise in Gegenwart eines Überschusses des als Reaktionspartner verwendeten Amins, eines tertiären Amins, beispielsweise von Pyridin oder Triethylamin, sowie anorganischer Basen, vorzugsweise Alkalihydroxide oder Alkalihydride.

11. Verwendung der Verbindungen nach Formel 1 gemäß einem der Ansprüche 1 bis 5 als therapeutische Wirkstoffe zur Herstellung von Arzneimitteln zur Behandlung von Erkrankungen, bei denen die Hemmung von TNF$\alpha$ therapeutisch nützlich ist.

12. Verwendung der Verbindungen nach Formel 1 gemäß einem der Ansprüche 1 bis 5 als therapeutische Wirkstoffe zur Herstellung von Arzneimitteln zur Behandlung von Erkrankungen, bei denen die Hemmung der Phosphodiesterase 4 therapeutisch nützlich ist.

13. Verwendung der Verbindungen nach Formel 1 gemäß den Ansprüchen 1 bis 5 als therapeutische Wirkstoffe zur Herstellung von Arzneimitteln zur Behandlung von Erkrankungen, die mit dem Wirken von Eosinophilen verbunden sind.

14. Verwendung der Verbindungen nach Formel 1 gemäß den Ansprüchen 1 bis 5 als therapeutische Wirkstoffe zur Herstellung von Arzneimitteln zur Behandlung von Erkrankungen, die mit dem Wirken von Neutrophilen verbunden sind.

15. Verwendung einer Verbindung nach einem der Ansprüche 1 bis 5 als Wirkstoff zur Herstellung eines Arzneimittels zur Behandlung oder/und Prophylaxe von Erkrankungen, bei denen eine Inhibition von TNF$\alpha$ nützlich ist, insbesondere von Gelenksentzündungen, Arthritis, rheumatoide Arthritis, arthritischen Erkrankungen, rheumatoider Spondylitis, Osteoarthritis, Osteoperose, Sepsis, septischem Schock, gram-negativer Sepsis, toxischem Schocksyndrom, Atemnotsyndrom, Asthma, chronischen pulmonalen Erkrankungen, Knochenresorptions-Krankheiten, Transplantat-Abstoßungsreaktionen, Autoimmunerkrankungen, Lupus erythematosus, Multipler Sclerose, Glomerulonephritis, Uveitis, Insulin abhängiger Diabetes mellitus, chronischer Demyelinisierung, von Viruserkrankungen, Virusinfektionen, Parasiteninfektionen, Malaria, Leishmaniasis, infektionsbedingtem Fieber, infektionsbedingten Muskelschmerzen, AIDS, Kachexien, Erkrankungen, die mit einer Inhibition der Phosphodesterase 4 behandelt werden können, Asthma, Erkrankungen, bei denen Eosinophile eine Rolle spielen, Asthma bronchiale, allergische Rhinits, allergische Konjunktivitis, atopische Dermatitis, Ekzeme, allergische Angiitis, durch Eosinophile vermittelte Entzündungen, eosinophile Fasciitis, eosinophile Pneumonie, PIE-Syndrom, Urtikaria, ulcerative Colitis, Crohn-Krankheit, proliferative Hauterkrankungen, Psoriasis, Keratosis, chronische obstruktive Lungenerkrankungen, Krankheiten, die durch Neuroprotektion behandelt werden können, senile Demenz, Alzheimer, Gedächtnisschwund, Parkinson, Depressionen, Schlaganfälle, Claudikatio intermittens, Prostata-Erkrankungen, benigne Prostata-Hyperplasie, Pollakisurie, Nocturie, Inkontinenz, Koliken, durch Harnsteine ausgelöste Koliken, männliche oder weibliche sexuelle Dysfunktionen sowie als Bronchiodilatatoren, zur Inhibition der Entstehung einer Arzneimittelabhängigkeit sowie zur Verringerung einer Toleranzentwicklung.

**16.** Arzneimittel, enthaltend eine oder mehrere Verbindungen gemäß den Ansprüchen 1 bis 5 neben üblichen physiologisch verträglichen Trägern und/oder Verdünnungsmitteln beziehungsweise Hilfsstoffen.

**17.** Verfahren zur Herstellung eines Arzneimittels nach Anspruch 16, **gekennzeichnet dadurch, dass** eine oder mehrere Verbindungen gemäß einem der Ansprüche 1 bis 5 mit gebräuchlichen pharmazeutischen Trägerstoffen und/oder Verdünnungsmitteln beziehungsweise sonstigen Hilfsstoffen zu pharmazeutischen Zubereitungen verarbeitet oder/und in eine therapeutisch anwendbare Form gebracht werden.

**18.** Verwendung von Verbindungen der allgemeinen Formel 1 gemäß einem der Ansprüche 1 bis 5 und/oder von pharmazeutischen Zubereitungen nach den Ansprüchen 16 oder 17 allein oder in Kombination untereinander oder in Kombination mit Trägerstoffen und/oder Verdünnungsmitteln beziehungsweise sonstigen Hilfsstoffen. Zur Herstellung eines Medikaments.

**19.** Verbindung nach Formel I gemäß einem der Ansprüche 1 bis 3, welche N-(3,5-Dichlorpyridin-4-yl)-[1-(4-fluorbenzyl)-7-azaindol-3yl]glyoxylsäureamid ist:

**Claims**

**1.** 7-Azaindoles of the formula 1

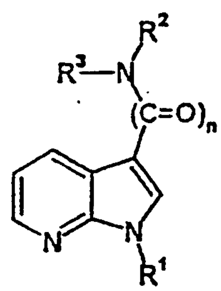

1

in which
n can be 1 or 2 and
**$R^1$** represents
$-C_1$ to $-C_{10}$ alkyl, linear or branched, unsubstituted or substituted one or more times with -OH, -SH, $-NH_2$, $-NHC_1$ to $-NHC_6$ alkyl, $-N(C_1$ to $C_6$-alkyl$)_2$, $-NHC_6$ to $-NHC_{14}$ aryl, $-N(C_6$ to $C_{14}$ aryl$)_2$, $-N(C_1$ to $C_6$ alkyl) ($C_6$ to $C_{14}$ aryl), $-NO_2$, -CN, -F, -Cl, -Br, -I, $-O-C_1$ to $-O-C_6$ alkyl, $-O-C_6$ to $-O-C_{14}$ aryl, $-S-C_1$ to $-S-C_6$ alkyl, $-S-C_6$ to $-S-C_{14}$ aryl, $-SO_3H$, $-SO_2C_1$ to $-SO_2C_6$ alkyl, $-SO_2C_6$ to $-SO_2C_{14}$ aryl, $-OSO_2C_1$ to $-OSO_2C_6$ alkyl, $-OSO_2C_6$ to $-OSO_2C_{14}$ aryl, -COOH, $-(CO)C_1$ to $-(CO)C_5$ alkyl, with mono-, bi- or tricyclic, saturated or monounsaturated or polyunsaturated carbocyclic compounds with 3 to 14 ring elements, with mono-, bi- or tricyclic saturated or monounsaturated or polyunsaturated heterocyclic groups with 5 to 15 ring elements and 1 to 6 hetero atoms, which preferably are N, 0 and S,
it being possible for the $C_6$ to $C_{14}$ aryl groups and the carbocyclic and heterocyclic substituents, in turn, to be unsubstituted or monosubstituted or polysubstituted with $R^4$,
$-C_2$ to $C_{10}$ alkenyl, monounsaturated or polyunsaturated, linear or branched, unsubstituted or monosubstituted or polysubstituted with -OH, -SH, $-NH_2$, $-NHC_1$ to $-NHC_6$ alkyl, $-N(C_1$ to $C_6$-alkyl$)_2$, $-NHC_6$ to $-NHC_{14}$ aryl, $-N(C_6$ to $C_{14}$ aryl$)_2$, $-N(C_1$ to $C_6$ alkyl) ($C_6$ to $C_{14}$ aryl), $-NO_2$, -CN, -F, -Cl, -Br, -I, $-O-C_1$ to $-O-C_6$ alkyl, $-O-C_6$ to $-O-C_{14}$ aryl, $-S-C_1$ to $-S-C_6$ alkyl, $-S-C_6$ to $-S-C_{14}$ aryl, $-SO_3H$, $-SO_2C_1$ to $-SO_2C_6$ alkyl, $-SO_2C_6$ to $-SO_2C_{14}$ aryl, $-OSO_2C_1$ to $-OSO_2C_6$ alkyl, $-OSO_2C_6$ to $-OSO_2C_{14}$ aryl, -COOH, $-(CO)C_1$ to $-(CO)C_5$ alkyl, with mono-, bi- or tricyclic, saturated or monounsaturated or polyunsaturated carbocyclic compounds with 3 to 14 ring elements, with mono-, bi- or

tricyclic saturated or monounsaturated or polyunsaturated heterocyclic groups with 5 to 15 ring elements and 1 to 6 hetero atoms, which preferably are N, O and S,
it being possible for the $C_6$ to $C_{14}$ aryl groups and the carbocyclic and heterocyclic substituents, in turn, to be unsubstituted or monosubstituted or polysubstituted with $R^4$,
**$R^2$** and **$R^3$** may be the same or different, it being possible for only one of the two to represent hydrogen and furthermore, $R^2$ and $R^3$ can represent $-C_1$-$C_5$ alkyl, unsubstituted or monosubstituted or polysubstituted with -OH, -SH, $-NH_2$, $-NHC_1$ to $-NHC_6$ alkyl, $-N(C_1$ to $C_6$-alkyl$)_2$, $-NO_2$, -CN, -F, -Cl, -Br-, -I, $-O-C_1$ to $-O-C_6$ alkyl, $-S-C_1$ to $-S-C_6$ alkyl, -phenyl, -pyridyl
-phenyl, unsubstituted or monosubstituted or polysubstituted with -OH, -SH, $-NH_2$, $-NHC_1$ to $-NHC_3$ alkyl, $-N(C_1$ to $C_3$-alkyl$)_2$, $-NO_2$, -CN, -COOH, $-COOC_1$ to $-COOC_3$ alkyl, -F, -Cl, -Br-, $-O-C_1$ to $-O-C_3$ alkyl, $-S-C_1$ to $-S-C_3$ alkyl,
-pyridyl, unsubstituted or monosubstituted or polysubstituted with $-NO_2$, -CN, -COOH, $-COOC_1$ to $-COOC_3$ alkyl, -Cl, -Br-, $-O-C_1$ to $-O-C_3$ alkyl, $-S-C_1$ to $-S-C_3$ alkyl,
as well as

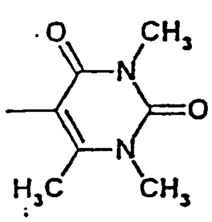
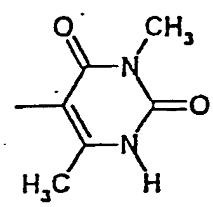
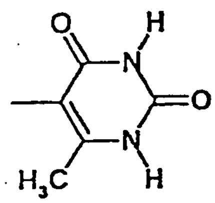
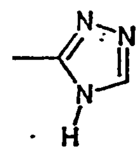

furthermore, the $-NR^2R^3$ group can represent together

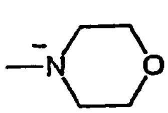
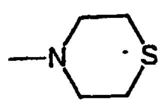
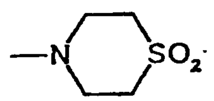
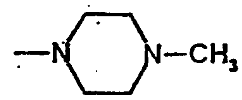

and
$R^4$ represents
-H, -OH, -SH, $-NH_2$, $-NHC_1$ to $-NHC_6$ alkyl, $-N(C_1$ to $C_6$ alkyl$)_2$, $NHC_6$ to $NHC_{14}$ aryl, $N(C_6$ to $C_{14}$ aryl$)_2$, $-N(C_1$ to $C_6$ alkyl$)(C_6$ to $C_{14}$ aryl), $-NHCOC_1$ to $-NHCOC_6$ alkyl, $-NO_2$, -CN, -COOH, $-COOC_1$ to $-COOC_6$ alkyl, $-(CO)C_1$ to $-(CO)C_6$ alkyl, $-(CS)C_1$ to $-(CS)C_6$ alkyl, -F, -Cl, -Br, -I, $-O-C_1$ to $-O-C_6$ alkyl, $-O-C_6$ to $-O-C_{14}$ aryl, $-S-C_1$ to $-S-C_6$ alkyl, $-S-C_6$ to $-S-C_{14}$ aryl, $-SOC_1$ to $-SOC_6$ alkyl, $-SO_2C_1$ to $-SO_2C_6$ alkyl,
with the proviso that when n = 1 there is no concurrent
$R_1 = -C_1$ to $C_6$ alkyl
$R_2$ = -H or $-C_1$ to $C_5$ alkyl and
$R_3$ =

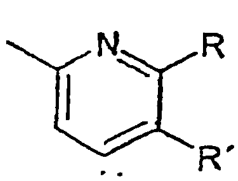
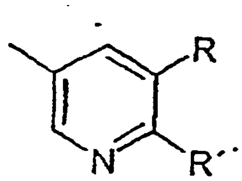
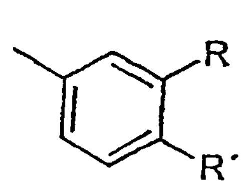

where R and R' are independently $-C_1$ to $C_6$ alkyl with or without single or multiple halogen substitution, $-C_2$ to $C_6$ alkenyl, $-C_3$ to $C_6$ cycloalkyloxy, $-C_3$ to $C_6$ cycloalkyl-$C_1$ to $C_6$ alkoxy, $-C_2$ to $C_6$ alkynyl, $-C_3$ to $C_6$ cycloalkyl, $-C_3$ to $C_6$ cycloalkyl-$C_1$ to $C_6$ alkyl, $-C_1$ to $C_6$ alkylthio, $-C_3$ to $C_6$ cycloalkylthio, $-C_3$ to $C_6$ cycloalkyl-$C_1$ to $C_6$ alkylthio, $-C_1$ to $C_6$ alkoxy, hydroxyl, halogen, nitro, $-CF_3$, $-C_2F_5$, $-OCF_3$, $-SCF_3$, $-SO_2CF_3$, $-SO_2F$, formyl, $-C_2$ to $C_6$ alkanoyl, cyano, substituted or unsubstituted phenyl or thienyl, $-NR''_2$, $-CONR''_2$, -COOR''
or

R + R' together are a 5-membered carbocycle or heterocycle,
and
R'' = -H or -$C_1$ to $C_6$ alkyl.

2. Physiologically tolerated salts of the compounds of formula 1 according to Claim 1, **characterized by** neutralization of the bases with inorganic or organic acids or neutralization of the acids with inorganic or organic bases or quaternization of tertiary amines to quaternary ammonium salts.

3. Compounds of formula 1 according to Claims 1 and 2 with an asymmetric carbon atom in the D form, the L form or in the form of D,L mixtures or, in the case of several asymmetric carbon atoms, the diastereoisomeric forms.

4. Compound of formula 1 according to any one of Claims 1 to 3 with n = 1, selected from the following compounds:

N-(4-pyridylmethyl)-1-cyclopropylmethyl-7-azaindol-3-carboxylic acid amide
N-(3,5-dichloropyridin-4-yl)-1-isobutyl-7-azaindol-3-carboxylic acid amide
N-(3,5-dichloropyridin-4-yl)-1-hexyl-7-azaindol-3-carboxylic acid amide
N-(3,5-dichloropyridin-4-yl)-1-cyclopropylmethyl-7-azaindol-3-carboxylic acid amide
N-(4-pyridylmethyl)-1-(4-fluorobenzyl)-7-azaindol-3-carboxylic acid amide
N-(3,5-dichloropyridin-4-yl)-1-(4-fluorobenzyl)-7-azaindol-3-carboxylic acid amide
N-(3,5-dichloropyridin-4-yl)-1-(4-methoxybenzyl)-7-azaindol-3-carboxylic acid amide
N-(4-pyridylmethyl)-1-(4-chlorobenzyl)-7-azaindol-3-carboxylic acid amide
1-(4-fluorobenzyl)-7-azaindol-3-carboxylic acid morpholide
N-(2,6-dichlorophenyl)-1-(2-methylpropen-3-yl)-7-azaindol-3-carboxylic acid amide and
N-(3,5-dichloropyridin-4-yl)-1-(4-pyridylmethyl)-7-azaindol-3-carboxylic acid amide.

5. Compound of formula 1 according to any one of Claims 1 to 3 with n = 2, selected from the following compounds:

N-(3,5-dichloropyridin-4-yl)-[1-(3-methoxybenzyl)-7-azaindol-3-yl]-glyoxylic acid amide
N-(4-pyridyl)-[1-(4-fluorobenzyl)-7-azaindol-3-yl]-glyoxylic acid amide hydrochloride
N-(3,5-dichloropyridin-4-yl)-[1-(4-fluorobenzyl)-7-azaindol-3-yl]-glyoxylic acid amide
N-(4-pyridyl)-[1-(4-chlorobenzyl)-7-azaindol-3-yl]-glyoxylic acid amide hydrochloride
N-(3,5-dichloropyridin-4-yl)-[1-(4-chlorobenzyl)-7-azaindol-3-yl]-glyoxylic acid amide
N-(3,5-dichloropyridin-4-yl)-[1-(4-methoxybenzyl)-7-azaindol-3-yl]-glyoxylic acid amide
N-(2,6-dichlorophenyl)-[1-(9-chlorobenzyl)-7-azaindol-3-yl]-glyoxylic acid amide
N-(4-carboxyphenyl)-[1-(4-fluorobenzyl)-7-azaindol-3-yl]-glyoxylic acid amide
N-(4-ethoxycarbonylphenyl)-[1-(4-fluorobenzyl)-7-azaindol-3-yl]-glyoxylic acid amide
N-(3,4-dimethoxyphenyl)-[1-(4-fluorobenzyl)-7-azaindol-3-yl]-glyoxylic acid amide
N-(3,5-dichloropyridin-4-yl)-[1-(4-methylbenzyl)-7-azaindol-3-yl]-glyoxylic acid amide
N-(3,5-dichloropyridin-4-yl)-[1-(4-hydroxybenzyl)-7-azaindol-3-yl]-glyoxylic acid amide
N-(3,5-dichloropyridin-4-yl)-[1-(3-hydroxybenzyl)-7-azaindol-3-yl]-glyoxylic acid amide
N-(3,5-dichloropyridin-4-yl)-(1-cyclopropylmethyl-7-azaindol-3-yl)-glyoxylic acid amide
N-(3,5-dichloropyridin-4-yl)-(1-hexyl-7-azaindol-3-yl)-glyoxylic acid amide
N-(3,5-dichloropyridin-4-yl)-(1-isobutyl-7-azaindol-3-yl)-glyoxylic acid amide
N-(3,5-dichloropyridin-4-yl)-[1-(2-methylpropen-3-yl)-7-azaindol-3-yl]-glyoxylic acid amide
N-(3,5-dichloropyridin-4-yl)-[1-(2-methoxyethyl)-7-azaindol-3-yl]-glyoxylic acid amide
N-(3,5-dichloropyridin-4-yl)-[1-(1-naphthylmethyl)-7-azaindol-3-yl]-glyoxylic acid amide
N-(3,5-dichloropyridin-4-yl)-[1-(4-pyridylmethyl)-7-azaindol-3-yl]-glyoxylic acid amide
N-(3,5-dichloropyridin-4-yl)-[1-(3,5-dimethylisoxazole-4-ylmethyl)-7-azaindol-3-yl]-glyoxylic acid amide
N,N-bis(2-methoxyethyl)-[1-(4-fluorobenzyl)-7-azaindol-3-yl]-glyoxylic acid amide
[1-(4-fluorobenzyl)-7-azaindol-3-yl]-glyoxylic acid morpholide
[1-(4-fluorobenzyl)-7-azaindol-3-yl]-glyoxylic acid (S,S-dioxo-thiomorpholide)
[1-(4-fluorobenzyl)-7-azaindol-3-yl]-glyoxylic acid (4-methylpiperazide)
N-(6-methyluracil-5-yl)-[1-(4-fluorobenzyl)-7-azaindol-3-yl]-glyoxylic acid amide
N-(3,6-dimethyluracil-5-yl)-[1-(4-fluorobenzyl)-7-azaindol-3-yl]-glyoxylic acid amide
N-(1,3,6-trimethyluracil-5-yl)-[1-(4-fluorobenzyl)-7-azaindol-3-yl]-glyoxylic acid amide and
N-(1,2,4-4H-triazol-3-yl)-[1-(4-fluorobenzyl)-7-azaindol-3-yl]-glyoxylic acid amide.

6. Method of preparing compounds of formula 1 according to any one of Claims 1 to 4 with n = 1, **characterized in**

**that** 7-azaindol-3-carboxylic acids are converted by means of acid chlorides into the analogous 7-azaindol-3-carboxylic acid chlorides and subsequently, by reaction with primary or secondary amines, converted to the inventive compounds of formula 1 with n = 1.

**7.** Preparation of compounds of formula 1 by the method according to Claim 6, **characterized by** the use of thionyl chloride or oxalyl chloride as acid chlorides for the synthesis of the 7-azaindol-3-carboxylic acid chlorides.

**8.** Preparation of compounds of formula 1 by the method according to Claim 6 or 7, **characterized by** the reaction of the 7-azaindol-3-carboxylic acid chlorides with primary or secondary amines in the presence of an auxiliary base, preferably in the presence of an excess of the amine used as reactant, of a tertiary amine, such as pyridine or triethylamine, as well as of inorganic bases, preferably alkali hydroxides or alkali hydrides.

**9.** Method for the preparation of compounds of formula 1 according to any one of Claims 1 to 3 and 5 with n = 2, **characterized in that** 7-azaindoles are converted into the analogous 7-azaindol-3-yl-glyoxylic acid chlorides with oxalyl chloride and subsequently into the compounds of formula 1, with n = 2, by reaction with primary or secondary amines.

**10.** Preparation of compounds of formula 1 by the method according to Claim 9, **characterized by** the reaction of 7-azaindol-3-yl-glyoxylic acid chlorides with primary or secondary amines in the presence of an auxiliary base, preferably in the presence of an excess of the amine used as the reactant, of a tertiary amine, such as pyridine or triethylamine, as well as of inorganic bases, preferably alkali hydroxides or alkali hydrides.

**11.** Use of compounds of formula 1 according to any one of Claims 1 to 5 as therapeutic active ingredients for the preparation of medicinal drugs for the treatment of diseases, for which the inhibition of TNF$\alpha$ is therapeutically useful.

**12.** Use of compounds of formula 1 according to any one of Claims 1 to 5 as therapeutic active ingredients for the preparation of medicinal drugs for the treatment of diseases for which the inhibition of phosphodiesterase 4 is therapeutically useful.

**13.** Use of the compounds of formula 1 according to Claims 1 to 5 as therapeutic active ingredients for the preparation of drugs for the treatment of diseases which are associated with the effects of eosinophiles.

**14.** Use of the compounds of formula 1 according to Claims 1 to 5 as therapeutic active ingredients for the preparation of drugs for the treatment of diseases which are associated with the effects of neutrophiles.

**15.** Use of a compound according to any one of Claims 1 to 5 as an active ingredient to produce a drug for treatment and/or prophylaxis of diseases where an inhibition of TNF$\alpha$ is beneficial, especially of joint inflammations, arthritis, rheumatoid arthritis, arthritic diseases, rheumatoid spondylitis, osteoarthritis, osteoperosis, sepsis, septic shock, Gram-negative sepsis, toxic shock syndrome, respiratory distress syndrome, asthma, chronic pulmonary diseases, bone resorption diseases, transplant rejection reactions, autoimmune diseases, lupus erythematosis, multiple sclerosis, glomerulonephritis, uveitis, insulin-dependent diabetes mellitus, chronic demyelination, of viral diseases, viral infections, parasite infections, malaria, leishmaniasis, infection-induced fever, infection-induced muscle pain, AIDS, cachexia, diseases which can be treated with an inhibition of phosphodiesterase 4, asthma, diseases associated with the effects of eosinophiles, bronchial asthma, allergic rhinitis, allergic conjunctivitis, atopic dermatitis, eczema, allergic angiitis, inflammations brought about by eosinophiles, eosinophilic fasciitis, eosinophilic pneumonia, PIE syndrome, urticaria, ulcerative colitis, Crohn's disease, proliferative skin diseases, psoriasis, keratosis, chronic obstructive lung diseases, diseases which can be treated by neuroprotection, senile dementia, Alzheimer's, memory loss, Parkinson's, depressions, strokes, intermittent claudications, prostata diseases, benign prostate hyperplasia, pollakiuria, nocturia, incontinence, colics, colics initiated by urinary calculus, male and female sexual dysfunctions and also as bronchodilators, for the inhibition of the development of drug dependence and also for reducing the development of tolerance.

**16.** Medicinal drugs, containing one or more compounds according to Claims 1 to 5 in addition to conventional, physiologically tolerated carriers and/or diluents or adjuvants.

**17.** Method for the preparation of a medicinal drug according to Claim 16, **characterized in that** one or more compounds according to any one of Claims 1 to 5, together with conventional pharmaceutical carrier materials and/or

diluents or other adjuvants, are processed into pharmaceutical preparations or/and brought into a therapeutically usable form.

**18.** Use of compounds of the general formula 1 according to any one of Claims 1 to 5 and/or of pharmaceutical preparations according to Claims 16 and 17 alone or in combination with one another or in combination with carrier materials and/or diluents or other adjuvants, for the preparation of a medicament.

**19.** Compound of formula I according to any one of Claims 1 to 3, which is N-(3,5-dichloropyridin-4-yl)-[1-(4-fluorobenzyl)-7-azaindol-3-yl]glyoxylic acid amide.

**Revendications**

**1.** 7-azaindoles de formule 1

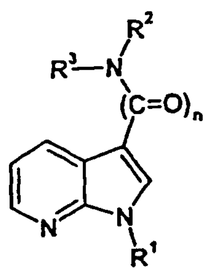

1

où
n peut être = 1 ou 2, et
$R^1$ représente
$-C_{1...10}$-alkyle, linéaire ou ramifié, non substitué ou substitué une ou plusieurs fois par -OH, -SH, $-NH_2$, $-NHC_{1...6}$-alkyle, $-N(C_{1...6}$-alkyle$)_2$, $-NHC_{6...14}$aryle, $-N(C_{6...14}$aryle$)_2$, $-N(C_{1...6}$alkyle$)(C_{6...14}$aryle), $-NO_2$, -CN, -F, -Cl, -Br, -I, $-O-C_{1...6}$alkyle, $-O-C_{6...14}$-aryle, $-S-C_{1...6}$-alkyle, $-S-C_{6...14}$-aryle, $-SO_3H$, $-SO_2C_{1...6}$-alkyle, $-SO_2C_{6...14}$-aryle, $-OSO_2C_{1...6}$-alkyle, $-OSO_2C_{6...14}$-aryle, -COOH, $-(CO)C_{1...5}$-alkyle, par des carbocycles à 3...14 chaînons cycliques mono-, bi- ou tricycliques saturés ou une ou plusieurs fois insaturés, par des hétérocycles mono-, bi- ou tricycliques saturés ou une ou plusieurs fois insaturés à 5...15 chaînons cycliques et 1...6 hétéroatomes qui sont de préférence N, O et S, où les groupes $C_{6...14}$aryle et les substituants carbocycliques et hétérocycliques peuvent quant à eux être non substitués ou substitués une ou plusieurs fois par $R^4$,
$-C_{2...10}$-alcényle, une ou plusieurs fois insaturé, linéaire ou ramifié, non substitué ou substitué une ou plusieurs fois par -OH, -SH, $-NH_2$, $-NHC_{1...6}$-alkyle, $-N(C_{1...6}$alkyle$)_2$, $-NHC_{6...14}$aryle, $-N(C_{6...14}$aryle$)_2$, $-N(C_{1...6}$alkyle$)(C_{6...14}$aryle), $-NO_2$, -CN, -F, -Cl, -Br, -I, $-O-C_{1...6}$-alkyle, $-O-C_{6...14}$-aryle, $-S-C_{1...6}$-alkyle, $-S-C_{6...14}$-aryle, $-SO_3H$, $-SO_2C_{1...6}$-alkyle, $-SO_2C_{6...14}$-aryle, $-OSO_2C_{1...6}$-alkyle, $-OSO_2C_{6...14}$-aryle, -COOH, $-(CO)C_{1...5}$-alkyle, par des carbocycles à 3...14 chaînons cycliques mono-, bi- ou tricycliques saturés ou une ou plusieurs fois insaturés, par des hétérocycles mono-, bi- ou tricycliques saturés ou une ou plusieurs fois insaturés à 5...15 chaînons cycliques et 1...6 hétéroatomes qui sont de préférence N, O et S, où les groupes $C_{6...14}$aryle et les substituants carbocycliques et hétérocycliques peuvent quant à eux être non substitués ou substitués une ou plusieurs fois par $R^4$,
$R^2$ et $R^3$ peuvent être identiques ou différents, où seul l'un des deux peut représenter l'hydrogène et $R^2$ et $R^3$ peuvent représenter en outre
$-C_{1...10}$-alkyle, non substitué ou substitué une ou plusieurs fois par -OH, -SH, $-NH_2$, $-NHC_{1...6}$-alkyle, $-N(C_{1...6}$-alkyle$)_2$, $-NO_2$, -CN, -F, -Cl, -Br, -I, $-O-C_{1...6}$-alkyle, $-S-C_{1...6}$-alkyle,
-phényle, -pyridyle
-phényle, non substitué ou substitué une ou plusieurs fois par -OH, -SH, $-NH_2$, $-NHC_{1...3}$-alkyle, $-N(C_{1...3}$-alkyle$)_2$, $-NO_2$, -CN, -COOH, $-COOC_{1...3}$alkyle, -F, -Cl, -Br, $-O-C_{1...3}$-alkyle, $-S-C_{1...3}$-alkyle,
-pyridyle, non substitué ou substitué une ou plusieurs fois par $-NO_2$, -CN, -COOH, $-COOC_{1...3}$alkyle,
-Cl, -Br, $-O-C_{1...3}$-alkyle, $-S-C_{1...3}$-alkyle,
ainsi que

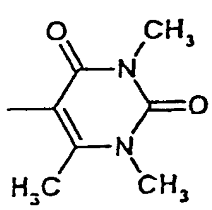
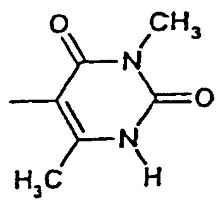
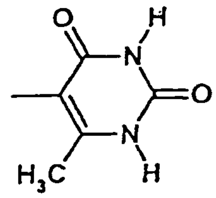
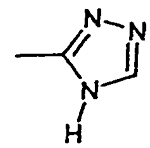

en outre, le groupe $-NR^2R^3$ peut représenter ensemble

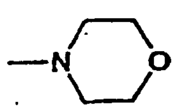
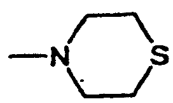
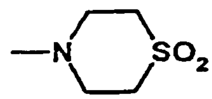
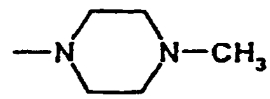

et

$R^4$ représente

-H, -OH, -SH, $-NH_2$, $-NHC_{1...6}$-alkyle, $-N(C_{1...6}\text{-alkyle})_2$, $-NHC_{6...14}$aryle, $-N(C_{6...14}\text{aryle})_2$, $-N(C_{1...6}\text{alkyle})(C_{6...14}\text{aryle})$, $-NHCOC_{1...6}$-alkyle, $-NO_2$, -CN, -COOH, $-COOC_{1...6}$-alkyle, $-(CO)C_{1...6}$-alkyle, $-(CS)C_{1...6}$-alkyle, -F, -Cl, -Br, -I, $-O-C_{1...6}$-alkyle, $-O-C_{6...14}$-aryle, $-S-C_{1...6}$-alkyle, $-S-C_{6...14}$-aryle, $-SOC_{1...6}$-alkyle, $-SO_2C_{1...6}$-alkyle,

avec la condition que, quand n = 1, on n'a pas simultanément

$R_1 = -C_{1...6}$-alkyle,

$R_2$ = -H ou $-C_{1...6}$-alkyle et

$R_3$ =

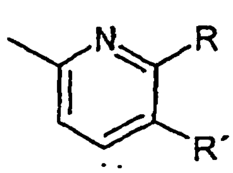
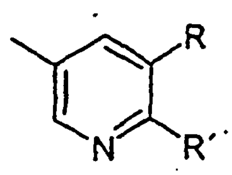
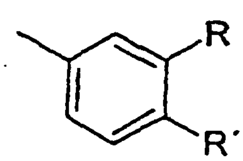

où R, R' sont indépendamment l'un de l'autre $-C_{1...6}$-alkyle, éventuellement substitué une ou plusieurs fois par halogène, $-C_{2...6}$-alcényle, $-C_{3...6}$-cycloalkyloxy, $-C_{3...6}$-cycloalkyl$C_{1...6}$-alcoxy, $-C_{2...6}$-alcynyle, $-C_{3...6}$-cycloalkyle, $-C_{3...6}$-cycloalkyl-$C_{1...6}$-alkyle, $-C_{1...6}$-alkylthio, $-C_{3...6}$-cycloalkylthio, $-C_{3...6}$-cycloalkyl$C_{1...6}$-alkylthio, $-C_{1...6}$-alcoxy, hydroxy-, halogène-, nitro-, $-CF_3$, $-C_2F_5$, $-OCF_3$, $-SCF_3$, $-SO_2CF_3$, $-SO_2F$, formyle-, $-C_{2...6}$-alcanoyle, cyano-, phényle ou thiényle éventuellement substitué, -NR"$_2$, -CONR"$_2$, -COOR"

ou

R + R' sont ensemble un carbocycle ou hétérocycle à 5 chaînons,

et

R" = -H ou $-C_{1...6}$-alkyle.

2. Sels physiologiquement acceptables des composés de formule 1 selon la revendication 1 **caractérisés par** la neutralisation des bases avec des acides inorganiques ou organiques ou par la neutralisation des acides avec des bases inorganiques ou organiques ou par la quaternisation d'amines ternaires en sels d'ammonium quaternaires.

3. Composés de formule 1 selon les revendications 1 et 2 avec un atome de carbone asymétrique sous la forme D, la forme L ou sous forme de mélanges D,L, ou, dans le cas de plusieurs atomes de carbone asymétriques, les formes diastéréoisomères.

4. Composés de formule 1 selon l'une des revendications 1 à 3 avec n = 1, choisis parmi les composés suivants :

N-(4-pyridylméthyl)-1-cyclopropylméthyl-7-azaindole-3-carboxamide,
N-(3,5-dichloropyridin-4-yl)-1-isobutyl-7-azaindole-3-carboxamide,
N-(3,5-dichloropyridin-4-yl)-1-hexyl-7-azaindole-3-carboxamide,
N-(3,5-dichloropyridin-4-yl)-1-cyclopropylméthyl-7-azaindole-3-carboxamide,
N-(4-pyridylméthyl)-1-(4-fluorobenzyl)-7-azaindole-3-carboxamide,
N-(3,5-dichloropyridin-4-yl)-1-(4-fluorobenzyl)-7-azaindole-3-carboxamide,
N-(3,5-dichloropyridin-4-yl)-1-(4-méthoxybenzyl)-7-azaindole-3-carboxamide,
N-(4-pyridylméthyl)-1-(4-chlorobenzyl)-7-azaindole-3-carboxamide, morpholide d'acide 1-(4-fluorobenzyl)-7-azaindole-3-carboxylique,
N-(2,6-dichlorophényl)-1-(2-méthylpropén-3-yl)-7-azaindole-3-carboxamide et
N-(3,5-dichloropyridin-4-yl)-1-(4-pyridylméthyl)-7-azaindole-3-carboxamide.

5. Composé de formule 1 selon l'une des revendications 1 à 3 avec n = 2, choisi parmi les composés suivants :

N-(3,5-dichloropyridin-4-yl)-[1-(3-méthoxybenzyl)-7-azaindol-3-yl]glyoxamide,
chlorhydrate de N-(4-pyridyl)-[1-(4-fluorobenzyl)-7-azaindol-3-yl]glyoxamide,
N-(3,5-dichloropyridin-4-yl)-[1-(4-fluorobenzyl)-7-azaindol-3-yl]glyoxamide,
chlorhydrate de N-(4-pyridyl)-[1-(4-chlorobenzyl)-7-azaindol-3-yl]-glyoxamide,
N-(3,5-dichloropyridin-4-yl)-[1-(4-chlorobenzyl)-7-azaindol-3-yl]glyoxamide,
N-(3,5-dichloropyridin-4-yl)-[1-(4-méthoxybenzyl)-7-azaindol-3-yl]glyoxamide,
N-(2,6-dichlorophényl)-[1-(4-chlorobenzyl)-7-azaindol-3-yl]glyoxamide,
N-(4-carboxyphényl)-[1-(4-fluorobenzyl)-7-azaindol-3-yl]glyoxamide,
N-(4-éthoxycarbonylphényl)-[1-(4-fluorobenzyl)-7-azaindol-3-yl]glyoxamide,
N-(3,4-diméthoxyphényl)-[1-(4-fluorobenzyl)-7-azaindol-3-yl]glyoxamide,
N-(3,5-dichloropyridin-4-yl)-[1-(4-méthoxybenzyl)-7-azaindol-3-yl]glyoxamide,
N-(3,5-dichloropyridin-4-yl)-[1-(4-hydroxybenzyl)-7-azaindol-3-yl]glyoxamide,
N-(3,5-dichloropyridin-4-yl)-[1-(3-hydroxybenzyl)-7-azaindol-3-yl]glyoxamide,
N-(3,5-dichloropyridin-4-yl)-[1-cyclopropylméthyl-7-azaindol-3-yl]glyoxamide,
N-(3,5-dichloropyridin-4-yl)-(1-hexyl-7-azaindol-3-yl]glyoxamide,
N-(3,5-dichloropyridin-4-yl)-(1-isobutyl-7-azaindol-3-yl]glyoxamide,
N-(3,5-dichloropyridin-4-yl)-[1-(2-méthylpropén-3-yl)-7-azaindol-3-yl]-glyoxamide,
N-(3,5-dichloropyridin-4-yl)-[1-(2-méthoxyéthyl)-7-azaindol-3-yl]glyoxamide,
N-(3,5-dichloropyridin-4-yl)-[1-(1-naphtylméthyl)-7-azaindol-3-yl]glyoxamide,
N-(3,5-dichloropyridin-4-yl)-[1-(4-pyridylméthyl)-7-azaindol-3-yl]glyoxamide,
N-(3,5-dichloropyridin-4-yl)-[1-(3,5-diméthylisoxazol-4-ylméthyl)-7-azaindol-3-yl]glyoxamide,
N,N-bis(2-méthoxyéthyl)-[1-(4-fluorobenzyl)-7-azaindol-3-yl]glyoxamide,
morpholide d'acide [1-(4-fluorobenzyl)-7-azaindol-3-yl]glyoxylique,
(S,S-dioxothiomorpholide) d'acide [1-(4-fluorobenzyl)-7-azaindol-3-yl]-glyoxylique,
(4-méthylpipérazide) d'acide [1-(4-fluorobenzyl)-7-azaindol-3-yl]glyoxylique,
N-(6-méthyluracil-5-yl)-[1-(4-fluorobenzyl)-7-azaindol-3-yl]glyoxamide,
N-(3,6-diméthyluracil-5-yl)-[1-(4-fluorobenzyl)-7-azaindol-3-yl]glyoxamide,
N-(1,3,6-triméthyluracil-5-yl)-[1-(4-fluorobenzyl)-7-azaindol-3-yl]glyoxamide et
N-(1,2,4-4H-triazol-3-yl)-[1-(4-fluorobenzyl)-7-azaindol-3-yl]glyoxamide.

6. Procédé de production de composés de formule 1 selon l'une des revendications 1 à 4 avec n = 1 **caractérisé en ce que** des acides 7-azaindole-3-carboxyliques sont convertis en les chlorures d'acides 7-azaindole-3-carboxyliques analogues au moyen de chlorures d'acide puis en les composés de formule 1 selon l'invention avec n = 1 par réaction avec des amines primaires ou secondaires.

7. Production de composés de formule 1 par le procédé selon la revendication 6 **caractérisée par** l'utilisation du chlorure de thionyle ou du chlorure d'oxalyle comme chlorures d'acide pour la synthèse des chlorures d'acide 7-azaindole-3-carboxylique.

8. Production de composés de formule 1 par le procédé selon la revendication 6 ou 7 **caractérisée par** la réaction des chlorures d'acide 7-azaindole-3-carboxylique avec des amines primaires ou secondaires en présence d'une base auxiliaire, de préférence en présence d'un excès de l'amine utilisée comme partenaire réactionnel, d'une amine tertiaire, par exemple la pyridine ou la triéthylamine, ainsi que de bases inorganiques, de préférence des hydroxydes alcalins ou des hydrures alcalins.

9. Procédé de production de composés de formule 1 selon l'une des revendications 1 à 3 et 5 avec n = 2 **caractérisé en ce que** des 7-azaindoles sont convertis en les chlorures d'acide 7-azaindol-3-yl-glyoxylique analogues avec le chlorure d'oxalyle puis sont convertis en les composés de formule 1 avec n = 2 par réaction avec des amines primaires ou secondaires.

10. Production de composés de formule 1 par le procédé selon la revendication 9 **caractérisée par** la réaction des chlorures d'acide 7-azaindol-3-yl-glyoxylique avec des amines primaires ou secondaires en présence d'une base auxiliaire, de préférence en présence d'un excès de l'amine utilisée comme partenaire réactionnel, d'une amine tertiaire, par exemple la pyridine ou la triéthylamine, ainsi que de bases inorganiques, de préférence des hydroxydes alcalins ou des hydrures alcalins.

11. Utilisation des composés de formule 1 selon l'une des revendications 1 à 5 comme principes actifs thérapeutiques pour la production de médicaments pour le traitement des maladies dans lesquelles l'inhibition de TNF$\alpha$ est thérapeutiquement utile.

12. Utilisation des composés de formule 1 selon l'une des revendications 1 à 5 comme principes actifs thérapeutiques pour la production de médicaments pour le traitement des maladies dans lesquelles l'inhibition de la phosphodiestérase 4 est thérapeutiquement utile.

13. Utilisation des composés de formule 1 selon les revendications 1 à 5 comme principes actifs thérapeutiques pour la production de médicaments pour le traitement des maladies qui sont liées à l'action des éosinophiles.

14. Utilisation des composés de formule 1 selon les revendications 1 à 5 comme principes actifs thérapeutiques pour la production de médicaments pour le traitement des maladies qui sont liées à l'action des neutrophiles.

15. Utilisation d'un composé selon l'une des revendications 1 à 5 comme principe actif pour la production d'un médicament pour le traitement et/ou la prophylaxie des maladies dans lesquelles une inhibition de TNF$\alpha$ est utile, en particulier des inflammations articulaires, de l'arthrite, de la polyarthrite rhumatoïde, des maladies arthritiques, de la spondylarthrite ankylosante, de l'arthrose, de l'ostéoporose, de la septicémie, du choc septique, de la septicémie gram-négative, du syndrome de choc toxique, du syndrome de détresse respiratoire, de l'asthme, des maladies pulmonaires chroniques, des maladie de résorption osseuse, des réactions de rejet des greffes, des maladies auto-immunes, du lupus érythémateux, de la sclérose en plaques, de la glomérulonéphrite, de l'uvéite, du diabète sucré insulinodépendant, de la démyélinisation chronique, des maladies virales, des infections virales, des infections parasitaires, de la malaria, de la leishmaniose, de la fièvre due à une infection, des douleurs musculaires dues à une infection, du SIDA, des cachexies, des maladies qui peuvent être traitées par une inhibition de la phosphodiestérase 4, de l'asthme, des maladies dans lesquelles les éosinophiles jouent un rôle, de l'asthme bronchique, de la rhinite allergique, de la conjonctivite allergique, de la dermatite atopique, de l'eczéma, de l'angéite allergique, des inflammations à médiation par les éosinophiles, de la fasciite à éosinophiles, de la pneumonie à éosinophiles, du syndrome de Löffler, de l'urticaire, de la colite ulcéreuse, de la maladie de Crohn, des maladies cutanées prolifératives, du psoriasis, de la kératose, des maladies pulmonaires obstructives chroniques, des maladies qui peuvent être traitées par neuroprotection, de la démence sénile, de la maladie d'Alzheimer, de la détérioration de la mémoire, de la maladie de Parkinson, des dépressions, des attaques, de la claudication intermittente, des maladies de la prostate, de l'hypertrophie de la prostate, de la pollakiurie, de la nycturie, de l'incontinence, des coliques, des coliques déclenchées par des calculs urinaires, des dysfonctionnements sexuels masculins et féminins ainsi que comme bronchodilatateurs, pour l'inhibition de la survenue d'une dépendances à des médicaments ainsi que pour réduire le développement d'une tolérance.

16. Médicament contenant un ou plusieurs composés selon les revendications 1 à 5 outre des supports et/ou diluants ou adjuvants physiologiquement acceptables courants.

17. Procédé de production d'un médicament selon la revendication 16 **caractérisé en ce qu'**un ou plusieurs composés selon l'une des revendications 1 à 5 sont mis sous forme de préparations pharmaceutiques et/ou mis sous une forme thérapeutiquement utilisable avec des supports et/ou diluants ou autres adjuvants pharmaceutiques courants.

18. Utilisation de composés de formule générale 1 selon l'une des revendications 1 à 5 et/ou de préparations pharmaceutiques selon les revendications 16 ou 17 seul(e)s ou en combinaison entre eux (elles) ou en combinaison avec des supports et/ou des diluants ou d'autres adjuvants pour la production d'un médicament.

**19.** Composé de formule I selon l'une des revendications 1 à 3 qui est le N-(3,5-dichloropyridin-4-yl)-[1-(4-fluorobenzyl)-7-azaindol-3-yl]glyoxamide.